# EUROPEAN PATENT APPLICATION

(11) **EP 4 349 967 A1**
(43) Date of publication of application: **10.04.2024**
(21) Application number: 22816484.4
(22) Date of filing: 02.06.2022
(51) Int. Cl.: C12N 5/074, C12N 5/0793, A61K 35/34, C07K 14/47, C07K 14/81, G01N 33/50

(54) **NOVEL PLURIPOTENT CELLS**

(30) Priority: 02.06.2021 KR 20210071679
(71) Applicant: Korea Research Institute of Bioscience and Biotechnology, Daejeon 34141 (KR); Daegu Gyeongbuk Institute Of Science and Technology, Daegu 42988 (KR)
(72) Inventor: KIM, Janghwan, Daejeon 34141 (KR); HA, Jeongmin, Daejeon 34141 (KR); KIM, Jong Kyoung, Pohang-si, Gyeongsangbuk-do 37673 (KR); LEE, Jeong Soo, Daejeon 34141 (KR); SON, Mi-Young, Daejeon 34141 (KR); CHUNG, Kyung-Sook, Daejeon 34141 (KR); NAM, Juhyeon, Daejeon 34141 (KR); BAEK, Areum, Daejeon 34141 (KR); JEON, Young Joo, Daejeon 34141 (KR)
(74) Representative: Dehns
(86) International application number: PCT/KR2022/007867
(87) International publication number: WO 2022/255825

(57) **Abstract**

The present invention relates to: a method for converting non-pluripotent cells into pluripotent cells; cells produced by the method; and uses thereof. The pluripotent cells of the present invention have low tumorigenicity while having excellent cell differentiation ability and pluripotency capable of differentiating into three germ layers, and can specifically separate pluripotent cells in the intermediate stage of reprogramming, and thus, it is possible to produce pluripotent cells having characteristics different from conventional induced pluripotent stem cells.

## Description

### Technical Field

The present invention relates to a method for converting non-pluripotent cells into pluripotent cells, cells converted by the method, and uses thereof.

### Background Art

Pluripotent stem cells may infinitely self-regenerate and differentiate into all adult cells. Meanwhile, the pluripotent stem cells are generally divided into embryonic stem cells, nuclear transfer embryonic stem cells, and induced pluripotent stem cells (iPSCs). The embryonic stem cells have a limitation of immune rejection reaction and an ethical limitation based on those essentially derived from embryos, and nuclear transfer embryonic stem cells, which are stem cells produced by transferring the nuclei of somatic cells to eggs, have the advantages of no immune rejection reaction and the possibility of mass-production, but have an ethical limitation due to the use of eggs and have the disadvantage of tumorigenicity.

Meanwhile, the iPSCs are stem cells made by inducing somatic cells, of which the differentiation has already been completed, to a cell stage before the differentiation through specific genetic manipulation. The advantage of the iPSCs is that it can produce stem cells having individual/disease-specific pluripotency. However, there are limitations in that the efficiency of producing iPSCs from differentiated somatic cells is only 1% or less, the efficiency of differentiating iPSCs to desired specific cells is also low, and there is tumorigenicity.

Accordingly, there is a need to develop a novel pluripotent cell having the pluripotency to differentiate into desired cells, wherein the tumorigenicity is significantly reduced or small compared to the tumorigenicity shown in the iPSCs, and having excellent cell differentiation ability, as well as a method for producing the novel pluripotent cell.

### Disclosure of the Invention

### Technical Problem

The present inventors have developed a pluripotent cell having lower tumorigenicity than that of iPSC and cell differentiation ability and pluripotency capable of differentiating into three germ layers while hardly expressing characteristic marker genes (*Nanog, Rex1,* and *Esrrb*) of the iPSC by treating a non-pluripotent cell, which has been already differentiated, with reprogramming factors, or making the non-pluripotent cell express reprogramming factors, thereby converting the fate of the cell, and thus have completed the present invention.

### Solution to Problem

In an embodiment of the present invention, the present invention provides a method for converting non-pluripotent cells into pluripotent cells, the method including inducing the non-pluripotent cells to express reprogramming factors, thereby overexpressing desmosome-related genes or epithelial cell differentiation-related genes.

In another embodiment of the present invention, the present invention provides pluripotent cells produced by the method for converting non-pluripotent cells into pluripotent cells.

In an embodiment of the invention, the present invention provides cells in which the expression of desmosome-related genes or epithelial cell differentiation-related genes is higher than the expression level of the corresponding gene of induced pluripotent stem cells (iPSCs), or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding gene in the iPSCs.

In another embodiment of the invention, the present invention provides a method for producing cells, the method including inducing the differentiation of cells in which the expression of desmosome-related genes or epithelial cell differentiation-related genes is higher than the expression level of the corresponding gene of induced pluripotent stem cells (iPSCs), or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding gene in the iPSCs.

In an embodiment of the invention, the present invention provides a composition for cell transplantation or biological tissue regeneration, the composition including, as active ingredients: the pluripotent cells; the cells in which the expression of desmosome-related genes or epithelial cell differentiation-related genes is higher than the expression level of the corresponding gene of induced pluripotent stem cells (iPSCs), or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding gene in the iPSCs; or the differentiation-induced cells.

In an embodiment of the invention, the present invention provides a method for evaluating the efficacy or toxicity of a substance to be tested, the method including bringing: the pluripotent cells; the cells in which the expression of desmosome-related genes or epithelial cell differentiation-related genes is higher than the expression level of the corresponding gene of induced pluripotent stem cells (iPSCs), or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding gene in the iPSCs; or the differentiation-induced cells, into contact with the substance to be tested.

In another embodiment of the present invention, the present invention provides a method for regenerating biological tissue by administering, to an individual, the pluripotent cells; the cells in which the expression of desmosome-related genes or epithelial cell differentiation-related genes is higher than the expression level of the corresponding gene of induced pluripotent stem cells (iPSCs), or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding genes in the iPSCs; or the differentiation-induced cells.

### Advantageous Effects of Invention

The pluripotent cells, formed by the method for converting non-pluripotent cells into pluripotent cells of the present invention, have low tumorigenicity while having excellent cell differentiation ability and pluripotency capable of differentiating into three germ layers, and can achieve a shortened production period compared to the conventional production process of induced pluripotent stem cells by specifically separating pluripotent cells in the intermediate stage of reprogramming.

### Brief Description of Drawings

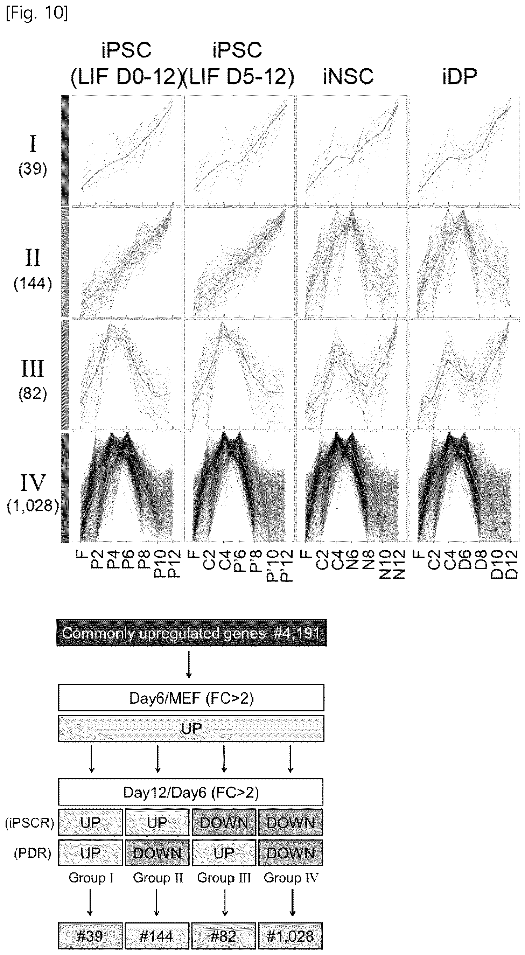

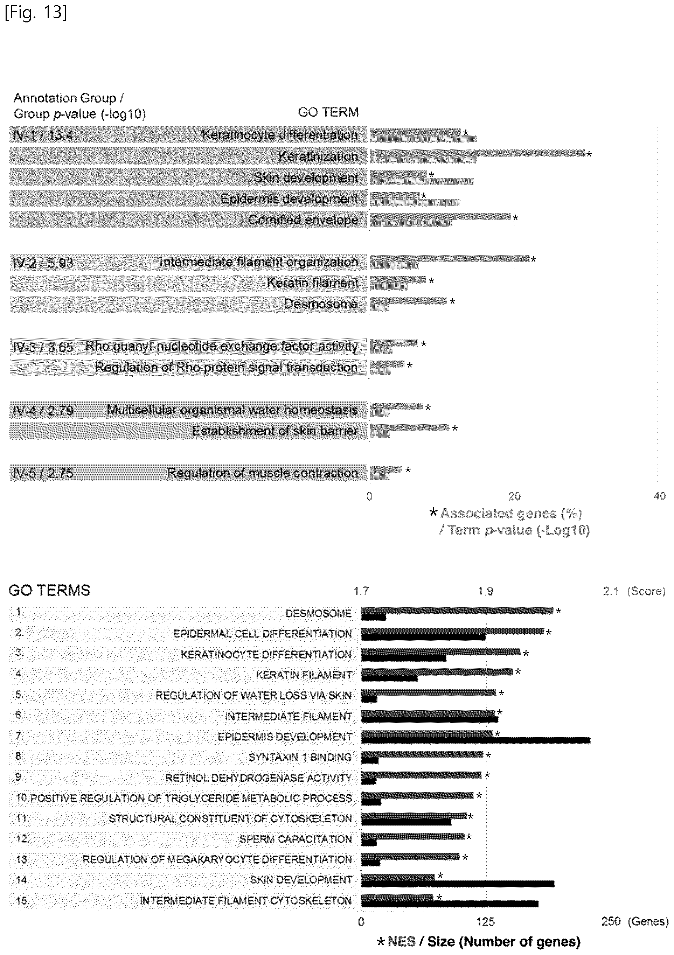

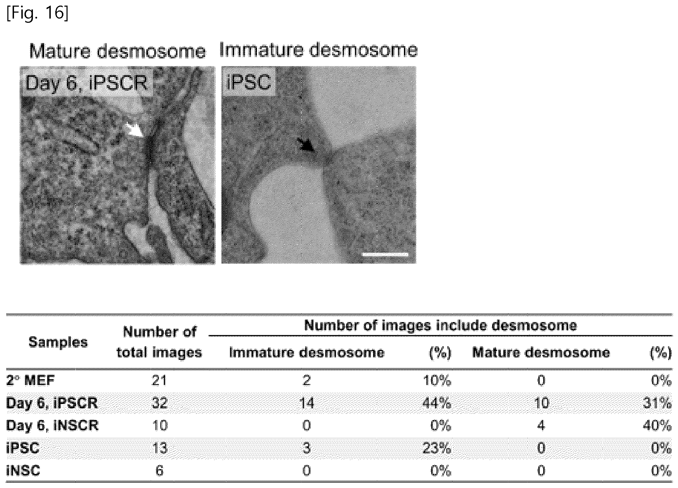

FIG. 1 is a schematic diagram of a process (iPSCR) of reprogramming 2°MEF into induced pluripotent stem cells (iPSCs), a process (PDR) of reprogramming 4F2A MEF into neural stem cells (iNSCs), and a process (PDR) of reprogramming 4F2A MEF into dopaminergic neural progenitor cells (iDPs).
FIG. 2 is a schematic diagram schematically illustrating the classification of cells in which total RNA is collected for each date during iPSCR and PDR processes.
FIG. 3 shows Nanog and SSEA-1 immunostaining photographs of iPSCs obtained via iPSCR, wherein the iPSCs are iPSCs supplied with LIF up to day 12 of iPSCR and supplied with LIF from day 5 to day 12 of iPSCR, a Pax6 and PLZF immunostaining photograph of iNSC obtained via PDR, and a FoxA2 immunostaining photograph of iDP obtained via PDR (scale bar: 200 µm).
FIG. 4 is an alkaline phosphatase (AP) staining photograph of iPSCs obtained via iPSCR, wherein the iPSCs are iPSCs supplied with LIF up to day 12 of iPSCR and iPSCs supplied with LIF from day 5 to day 12 of iPSCR.
FIG. 5 shows a phase-contrast photograph of iPSCs obtained via iPSCR, wherein the iPSCs are iPSCs supplied with LIF up to day 12 of iPSCR and supplied with LIF from day 5 to day 12 of iPSCR, a phase-contrast photograph of iNSCs obtained via PDR, and a phase-contrast photograph of iDPs obtained via PDR (scale bar: 200 µm).

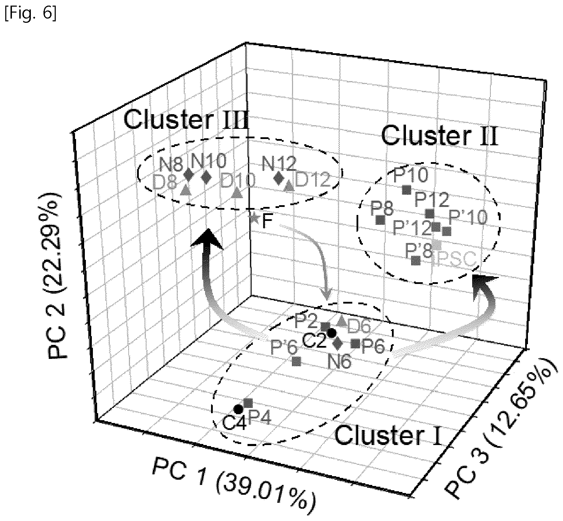

FIG. 6 shows principal component analysis (PCA) results for microarray data of iPSCs obtained via iPSCR, iNSCs obtained via PDR, and iDPs obtained via PDR. The individual numbers indicate the date when RNA was harvested after reprogramming, F refers to fibroblasts, C refers to intermediate cells formed under the condition of no LIF, P refers to the iPSCR condition in which LIF is supplied to day 12 of the culture, P' refers to the iPSCR condition in which LIF is supplied from day 5 to day 12 of the culture, N refers to the PDR condition of reprogramming with iNSCs, and D refers to the PDR condition of reprogramming with iDPs.
FIG. 7 shows a Pearson correlation matrix showing the microarray data hierarchical clustering of iPSCs obtained via iPSCR, iNSCs obtained via PDR, and iDPs obtained via PDR.
FIG. 8 shows a heat map showing the gene expression of the cells (P6, P'6, N6, and D6) on day 6 of the iPSCR and PDR processes and the cells (P12, P' 12, N12, and D12) on day 12 of the iPSCR and PDR processes.
FIG. 9 shows expression patterns of genes that changes during the iPSCR process.
The top of FIG. 10 shows individual expression patterns of iPSCs obtained via iPSCR wherein the iPSCs are iPSCs supplied with LIF up to day 12 of iPSCR, iPSCs supplied with LIF from day 5 to day 12 of iPSCR, iNSCs obtained via PDR, and iDPs obtained via PDR, wherein the number of genes for each group is indicated on the left side, the numerical values are normalized based on the average expression values of each gene and shown as a line plot, and a thick line shows the average value of gene expression (the top of FIG. 10). In addition, the bottom of FIG. 10 shows a selection process of DEG as flow-chart.

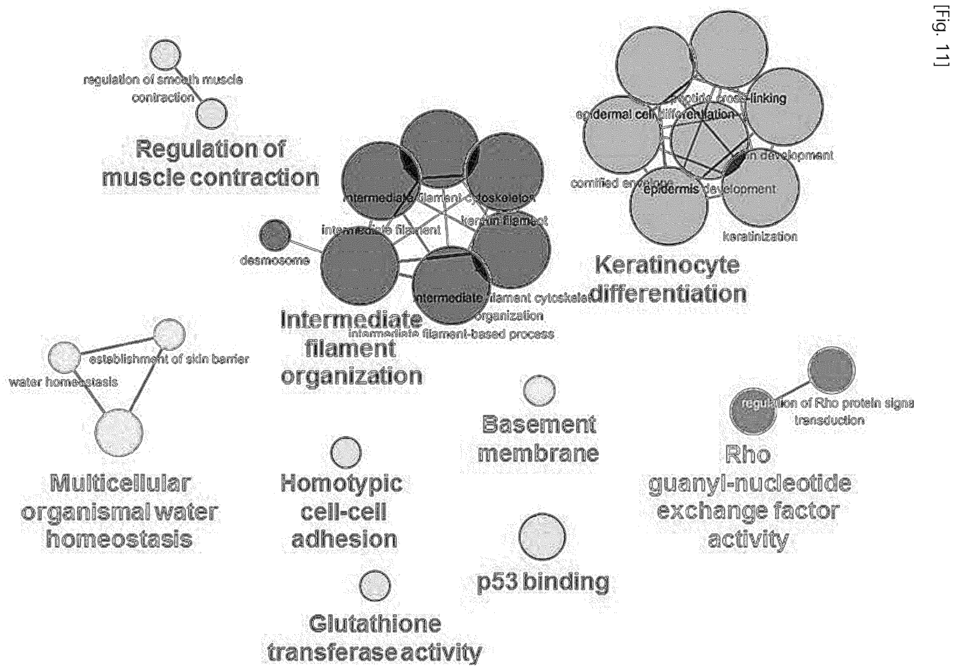

FIG. 11 shows the GO term of 244 common genes visualized by ClueGO with a network-based analysis. Major GO term was indicated as a node (p-value < 0.08), and a line connected between nodes was generated based on a predefined kappa score (> 0.4). Each node size represents p-value and is indicated in the order of p-value < 0.001; 0.001 < p-value < 0.05; and 0.05 < p-value < 0.01.

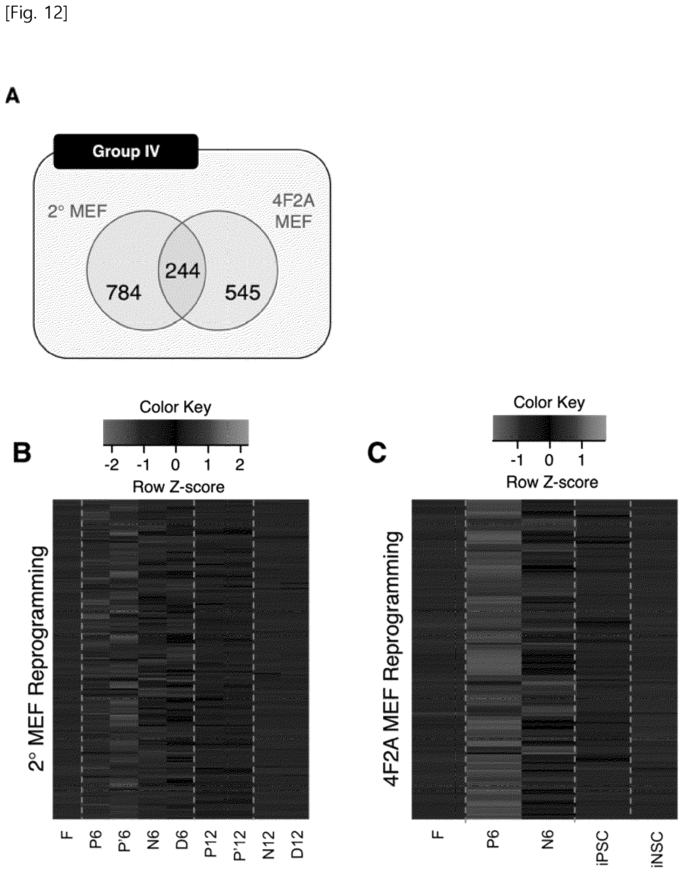

FIG. 12A shows a Venn diagram of the Group IV genes of 2°MEF and 4F2AMEF. FIG. 12B shows a heat map showing the expression of 244 genes of 2°MEF in the iPSCR process, and FIG. 12C shows a heat map showing the expression of 244 genes of 4F2AMEF in the PDR process of 4F2AMEF.
The top of FIG. 13 shows the top five groups as a result of the ClueGO analysis, the left panel shows representative GO term and ranks of each group, and the right panel shows gene percentages related to specific GO term and term p-value. The bottom of FIG. 13 shows the top 15 GO terms by GSEA, wherein GSEA was performed using transcriptomes of intermediate cells (ICs) obtained on day 6 of 2°MEF and 4F2A MEF reprogramming, transcriptomes of 2°MEF and 4F2A MEF, and transcriptomes of P12, P'12, N12 and D12, which were the endpoint cells on day 12 of 2°MEF and 4F2A MEF reprogramming. The bar represents normalized enrichment scores (NES), which are normalized higher scores, and the number of genes included in each GO term.

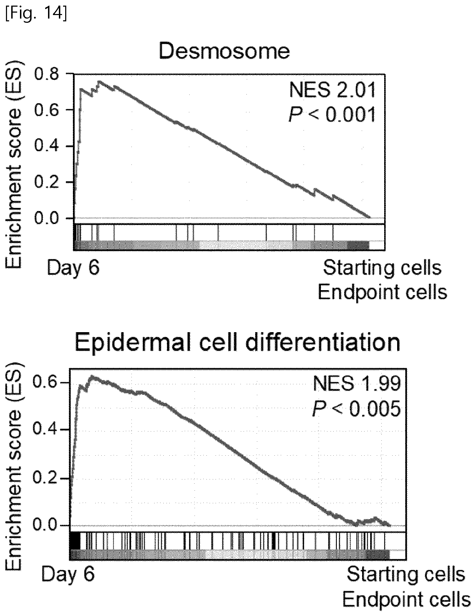

FIG. 14 is a graph showing the NES results of desmosome and epithelial cell differentiation according to gene set enrichment analysis.

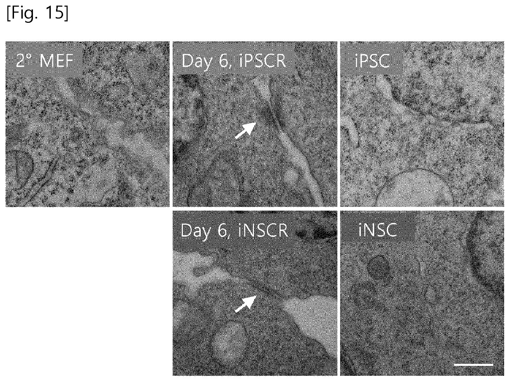

FIG. 15 shows transmission electron microscope photographs of cells on day 6 of 2°MEF, iPSCR and iNSCR, iPSCs and iNSCs, wherein white arrows indicate mature desmosomes (scale bar: 0.5 µm).
The top of FIG. 16 shows transmission electron microscope photographs of a cellular junction region of cell on day 6 of iPSCR and iNSCR, wherein a white arrow indicates a mature desmosome and a black arrow indicates an immature desmosome (scale bar: 0.5 µm). The bottom of FIG. 16 is a table showing the number of mature desmosomes and immature desmosomes for each sample.

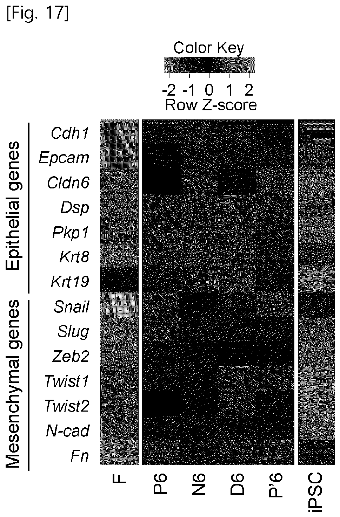

FIG. 17 is a graph showing the expression of an epithelial marker and a mesothelial marker as a heat map.

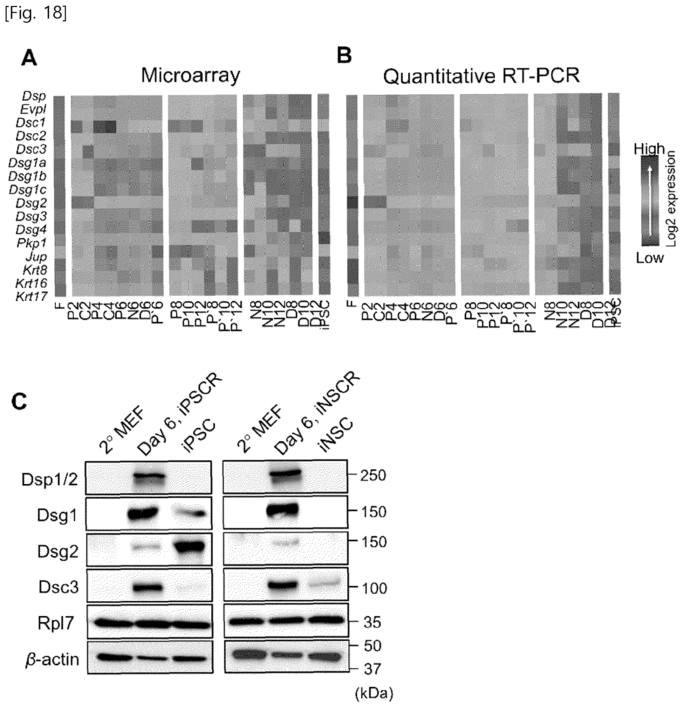

FIG. 18A is a graph showing, as a heat map, the results of microarray analysis of desmosome-related genes in the process of reprogramming 2°MEF, and FIG. 18B is a graph showing, as a heat map, the results of quantitative RT-PCR of desmosome-related genes in the process of reprogramming 2°MEF, and each numerical value is normalized after log conversion. FIG. 18C is a western blot analysis photograph of desmosome-related genes in the process of reprogramming 2° MEF, wherein Rpl7 and β-actin were used as loading control groups.

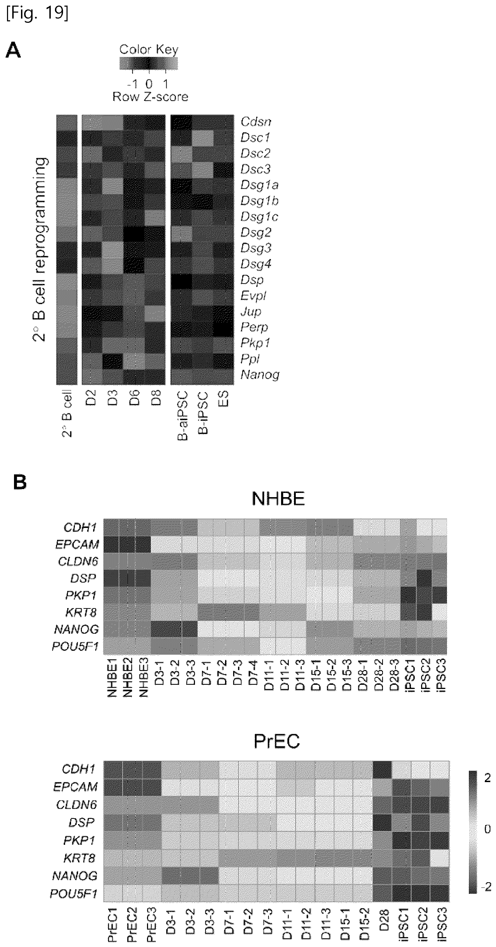

FIG. 19A is a graph showing, as a heat map, the expression of desmosome-related genes in the process of reprogramming 2° B cells analyzed using the published microarray data (GSE52397). FIG. 19B is a graph showing, as a heat map, patterns in which epithelial identity and the expression of desmosome-related genes and pluripotency-related genes are changed in the process of reprogramming human bronchial epithelial cells (NHBE) and prostate epithelial cells (PrEC).

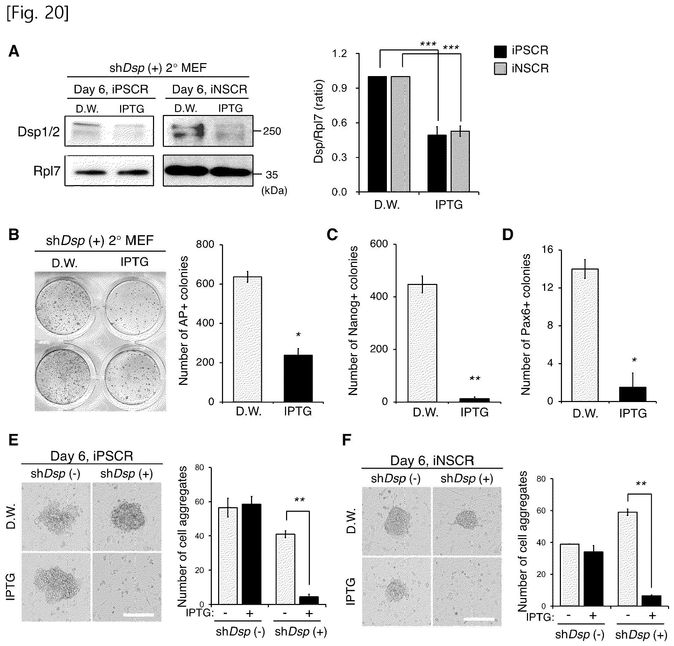

FIG. 20Ais a western blot result of the expression of Dsp and Rpl7 in the processes of iPSCR and PDR for 2° MEF classified as sh*D*sp-GFP positive. The expression of sh*D*sp is induced by the treatment of IPTG. The bar graph shows the quantitative expression of Dsp, and each sample was obtained on day 6 of iPSCR and iNSCR. Rpl7 was used as a loading control group (p-value < 0.0001), D.W. refers to distilled water, and IPTG represents isopropyl β-D-1-thiogalactopyranoside. FIG. 20B shows an AP staining image and an AP-positive colony quantitative analysis result (p-value = 0.0112) for the entire well on day 12 of reprogramming, and FIG. 20C shows a Nanog-positive colony quantitative analysis result (p-value = 0.0056) for each distilled water- or IPTG-treated group on day 12 of reprogramming. FIG. 20D shows a Pax6-positive colony quantitative analysis result (p-value = 0.0202) for each distilled water- or IPTG-treated group on day 12 of reprogramming. FIG. 20E shows a phase-contrast image of a cell aggregate on day 6 of iPSCR and a quantitative analysis result of the cell aggregate (sh*D*sp (-), p-value = 0.80; sh*D*sp (+), p-value = 0.0047), and FIG. 20F shows a phase-contrast image of a cell aggregate on day 6 of iNSCR and a quantitative analysis result of the cell aggregate (sh*D*sp (-), p-value = 0.34; sh*D*sp (+), p-value = 0.0015). In addition, the error bar of FIG. 20A means ± SEM (A), the error bars of FIGS. 20B to 20F mean ± SD, the statistical analysis was performed using two-tailed, paired Student's *t* test, and p-values < 0.05 were considered significant, * indicates p-value < 0.05, and ** indicates p-value < 0.01.

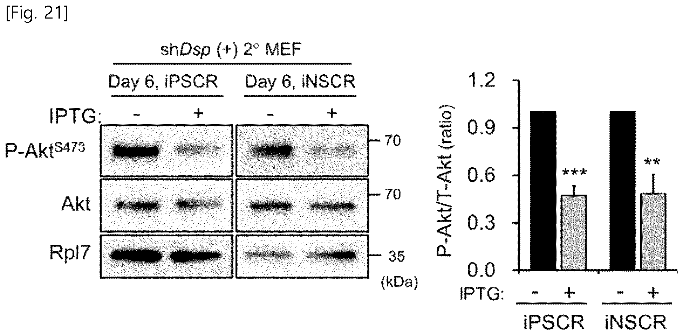

FIG. 21 shows a western blot image on day 6 of iPSCR and iNSCR depending on the presence or absence of Dsp knockdown, and a quantitative analysis graph of phospho-Akt and total Akt (iPSCR, p-value = 0.0002; iNSCR, p-value = 0.0055).

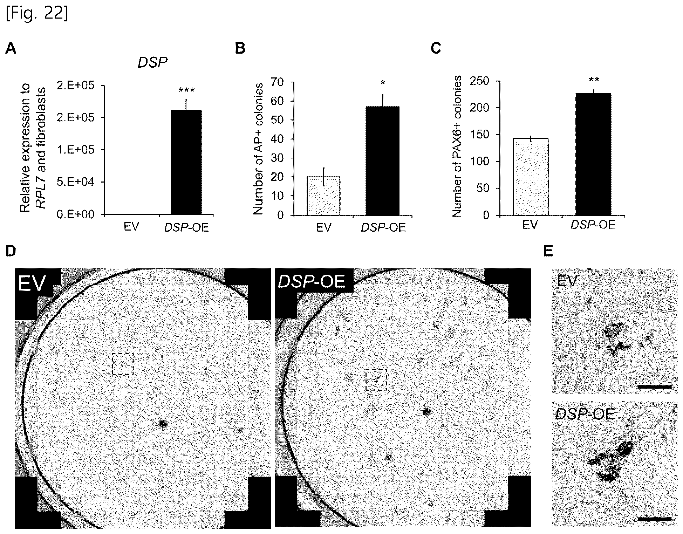

FIG. 22A shows the results of reverse-transcription qPCR (RT-qPCR) analysis of DSP expression according to the overexpression (*DSP*-OE) of DSP in human fibroblasts, the results were normalized with the expression of RPL7, and EV indicates an empty vector (p-value < 0.0001). FIG. 22B shows the results of quantitative analysis of the number of AP-positive colonies on day 16 of iPSCR of DSP-overexpressed fibroblasts (p-value = 0.0102). FIG. 22C shows the results of quantitative analysis of the number of PAX6-positive colonies on day 16 of iNSCR of DSP-overexpressed fibroblasts (p-value = 0.0053). FIG. 22D is a representative AP staining image of AP-positive colonies on day 16 of iPSCR of DSP-overexpressed fibroblasts, and FIG. 22E is an enlarged image of the box region of FIG. 22D. The scale bar is 100 µm, the error bar of FIG. 22A is ± SEM (A), the error bar of FIG. 22B is ± SE, and the error bars of FIGS. 22C and 22D are ± SD. The statistical analysis was performed using two-tailed, paired Student's *t* test, and p-values < 0.05 were considered significant, * indicates p-value < 0.05, ** indicates p-value < 0.01, and *** indicates p-value < 0.001.

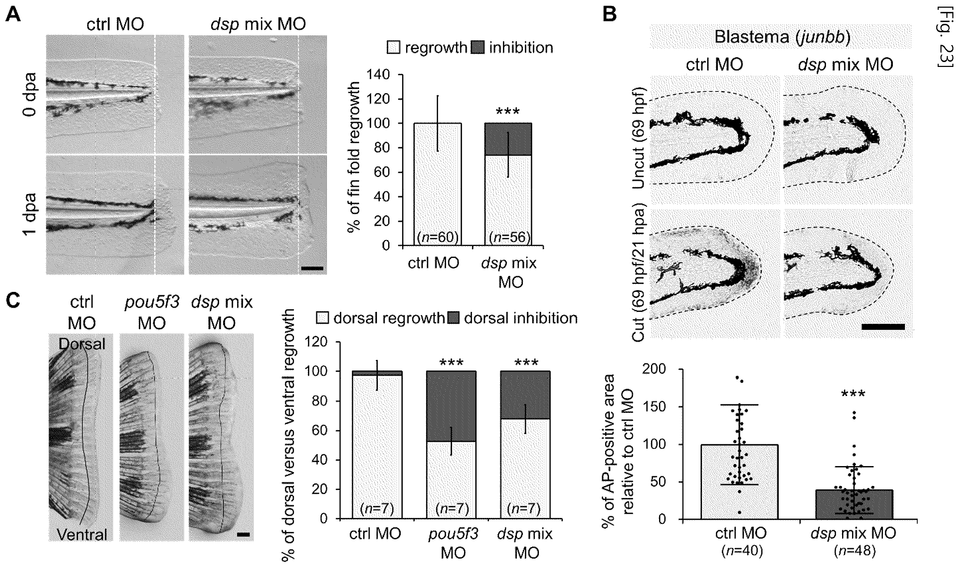

FIG. 23A is a phase-contrast image of fin-fold after the amputation of zebrafish fins, in which the fin-fold amputation was done at 2pf and photographed 24 hours later. In the graph, the light gray indicates the percentage of 1 day post-amputation (dpa) vs. 0 dpa fin fold outgrowth, and the deep gray indicates 1 dpa vs. 0 dpa fin-fold inhibition. The scale bar is 100 µm (n = 60, 56 embryos, p-value < 0.0001). MO stands for morpholino, and hpa stands for hours post-amputation. FIG. 23B shows a phase-contrast image of Whole-mount *in situ* hybridization analysis of fin-fold in the regenerated fins by comparing control MO or dsp mix MO, and the analysis results of the expression of blastema marker gene (*junbb*). The uncut fin was used as a control group, the dotted line indicates the boundary of the fin-fold, the scale bar is 100 µm, and hpf stands for hours post-fertilization (n = 40, 48 embryos, p-value < 0.0001). FIG. 23C shows a phase-contrast image and fluorescence merged image of the adult fin after the injection of control MO, pou5f3 MO (positive control) and dsp mix MO and electroporation. The injection of the materials was made on the dorsal half of the 2dpa blastema and was photographed 24 hours later, and the graph shows the growth of the dorsal fin vs. ventral fin (light gray) and the mean inhibition of dorsal fin vs. ventral fin (dark gray). The line in the left image of FIG. 23C indicates a fin of 2 dpa, and the scale bar is 500 µm (n = 7 zebrafish, p-value < 0.0001). In addition, the error bar was ± SD, two-tailed, paired Student's *t* test was used for statistical analysis, and when *P* values < 0.05 were considered significant (***p-value < 0.001).

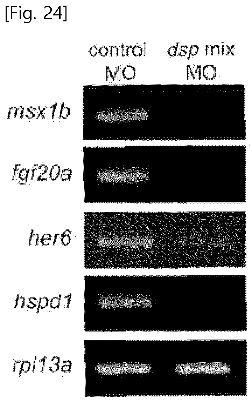

FIG. 24 shows a decrease in the expression of the gene marker related to the blastema cells according to the inhibition of the *Dsp* gene expression, confirmed by RT-PCR, and *Rpl13a* was used as an internal control.

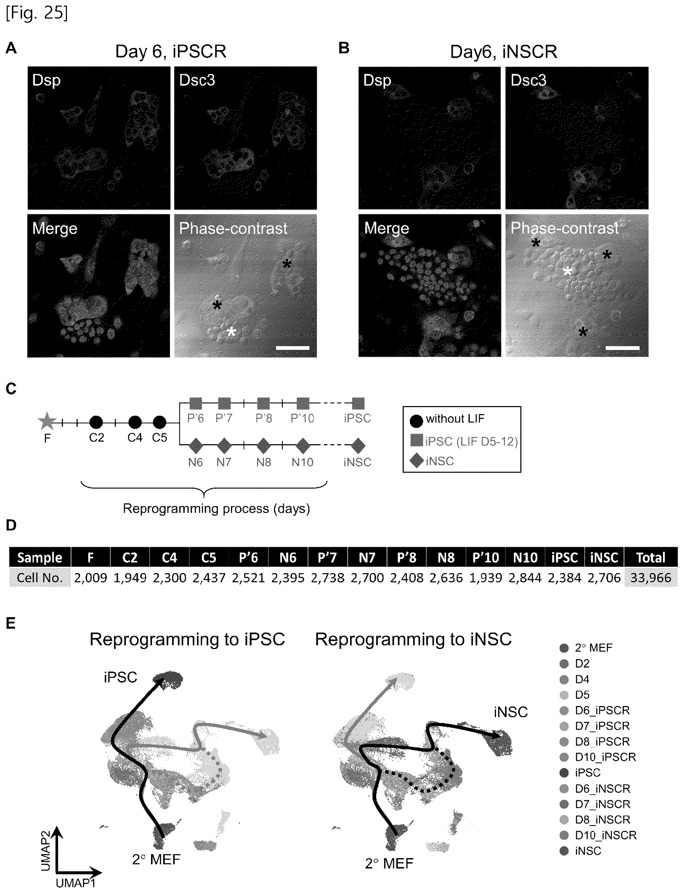

FIGS. 25A and 25B show immunostaining images and phase-contrast images for Dsp and Dsc3 of cells on day 6 of iPSCR and iNSCR under normal conditions, respectively. Black asterisks indicate Dsp- and Dsc3-positive cells in colonies, and white asterisks indicate Dsp- and Dsc3-negative cells (scale bar: 50 µm). FIG. 25C is a schematic diagram of sampling for scRNA-seq. FIG. 25D shows the number of single cell library for each reprogramming date. FIG. 25E shows a branch reprogramming trajectory by Slingshot as UMAP plot of cells in the process of iPSCR or iNSCR, and the shading is indicated differently for each cell harvesting time point, and a dotted line indicates a path through which reprogramming fails.

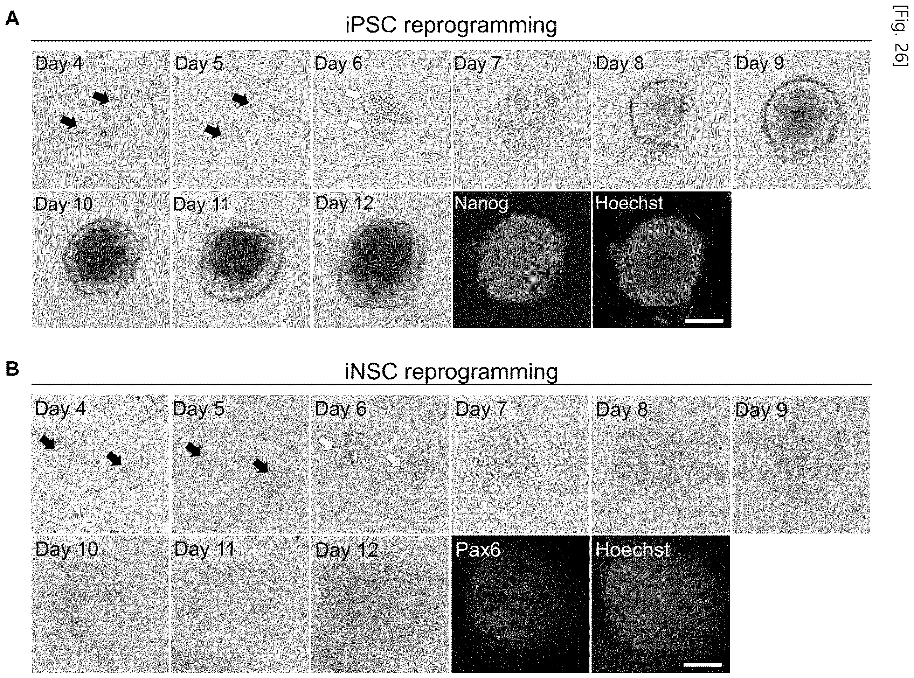

FIG. 26 shows photographs taken through a microscope of intermediate cells induced through the temporary expression of reprogramming factors, induced pluripotent stem cells (iPSCs) and neural stem cells (iNSCs) formed therefrom.

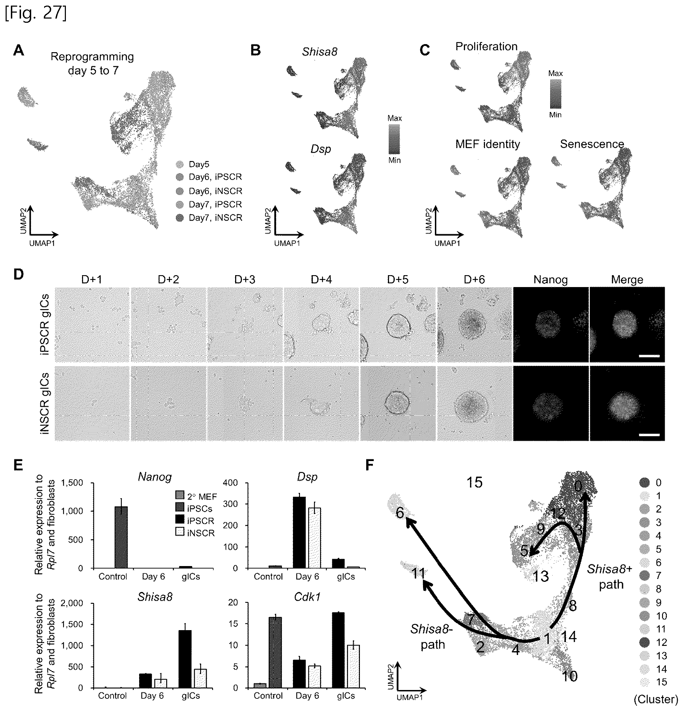

FIG. 27A shows, as UMAP plot, scRNA-seq data of cells from day 5 to day 7 of iPSCR and iNSCR. FIG. 27B shows the signature score distribution of *shisa8* and *Dsp* genes, and FIG. 27C shows the signature score distribution of an identity gene set that defines dIC and gIC in UMAP (scale bar shows the minimum to maximum signature scores that are shown in the entire reprogramming process). FIG. 27D shows phase-contrast images and immunostaining images of re-attachment of gIC from day 6 of iPSCR or iNSCR, and the immunostaining was performed on Nanog 6 days after re-attachment (D+6) (scale bar: 100 µm). FIG. 27E shows the reverse-transcription qPCR results of Nanog, Dsp, Shisa8, and Cdk1 genes, wherein 2°MEF was used as a negative control, iPSCs were used as a positive control, the sample on day 6 included all cells on day 6, and the gIC was separated on day 6. FIG. 27F shows reprogramming pathways expected depending on the presence or absence of Shisa8 expression.

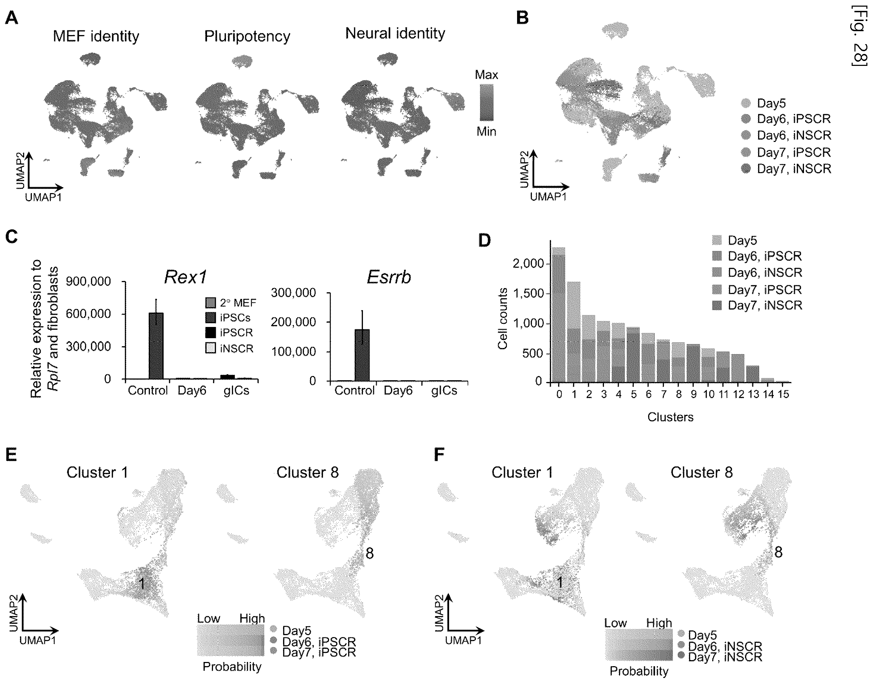

FIG. 28A shows UMAP plots of cells in the process of iPSC or iNSC for each gene set signature score. FIG. 28B shows UMAP plots of cells on day 5 and day 7, and the shading was changed for each harvesting time point. FIG. 28C shows the results of RT-qPCR analysis of the pluripotent marker, wherein 2°MEF was used as a negative control, iPSCs were used as a positive control, the sample on day 6 included all cells on day 6, and the gIC was separated on day 6. The error bar is ± SE. FIG. 28D shows, as a bar plot, the total number of cells having different shades according to the harvesting time point for each cluster. FIG. 28E shows a UMAP plot of cells that highlight the descendants of Cluster 1 and Cluster 8 in the process of iPSCR, FIG. 28F shows a UMAP plot of cells that highlight the descendants of Cluster 1 and Cluster 8 in the process of iNSCR, and the color and intensity show the descendant probabilities estimated by WOT and the harvesting time point.

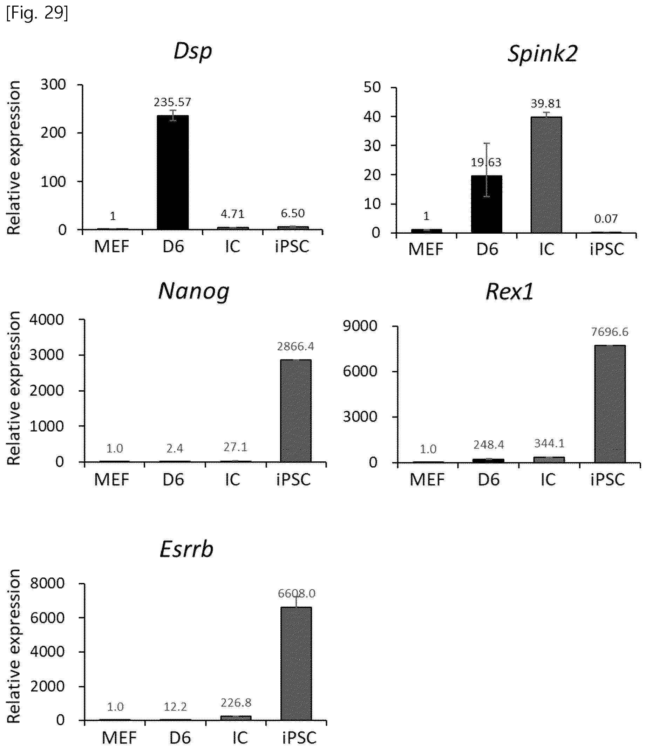

FIG. 29 shows graphs obtained by comparing the expression of the gene markers of intermediate cells (ICs) and induced pluripotent stem cells (iPSCs).

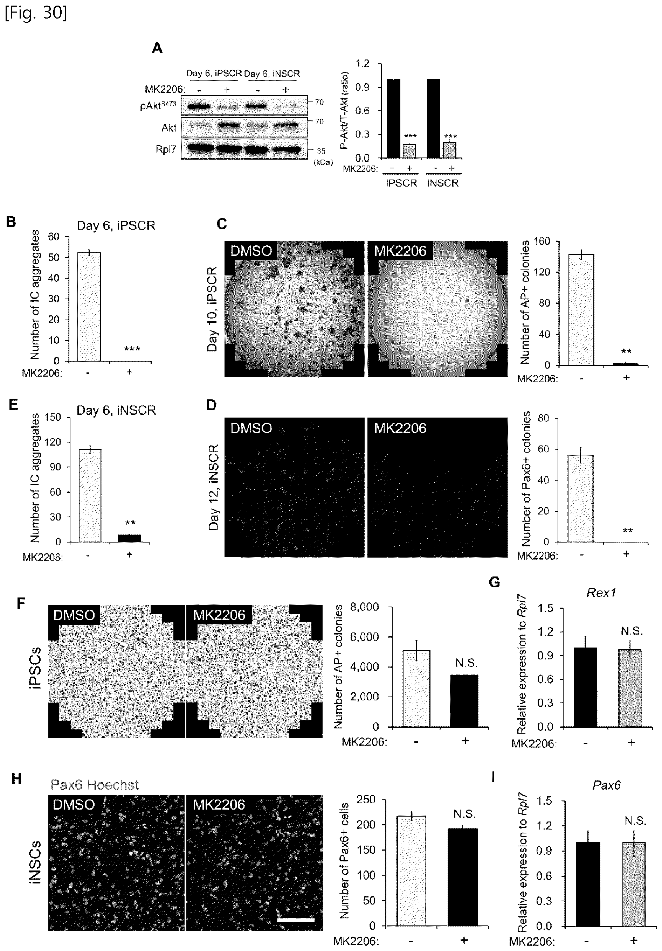

FIG. 30A shows western blot images and quantitative analysis results of phospho-Akt and total Akt on day 6 of iPSCR and iNSCR depending on the presence or absence of MK2206 (Akt inhibitor). Rpl7 was used as a loading control, p value for iPSCR was 0.00008, and p value for iNSCR was 0.00095. FIG. 30B is a quantitative analysis result of the IC aggregates on day 6 of iPSCR depending on the presence or absence of MK2206 (p value = 0.0009), and FIG. 30C is a quantitative analysis result of the IC aggregates on day 6 of iNSCR depending on the presence or absence of MK2206 (p value = 0.0019). FIG. 30D shows overall well images and a quantitative analysis result for AP-positive colonies among colonies on day 12 of iPSCR depending on the presence or absence of MK2206 (p value = 0.0020), and FIG. 30E shows overall well images and a quantitative analysis result for Pax6-positive colonies among colonies on day 12 of iNSCR depending on the presence or absence of MK2206 (p value = 0.0020). FIG. 30F shows overall well images and a quantitative analysis result for AP-positive colonies in iPSCs depending on the presence or absence of MK2206, and FIG. 30G shows the RT-qPCR analysis result of *Rex1,* which is a pluripotency-related marker in iPSCs depending on the presence or absence of MK2206 (p value = 0.13). FIG. 30H shows fluorescence images and a quantitative analysis result of Pax6-positive cells in iNSCs depending on the presence or absence of MK2206 (scale bar: 100 µm, p value = 0.15). FIG. 30I shows the RT-qPCR analysis result of Pax6, which is a neural stem cell marker in iNSCs depending on the presence or absence of MK2206. The error bar in FIG. 30Ais ± SEM, the error bars in FIGS. 30B to 30F and FIG. 30H are ± SD, and the error bars in FIGS. 30G and 30I are ± SE. All statistical analyses were performed using two-tailed, paired Student's *t* test.

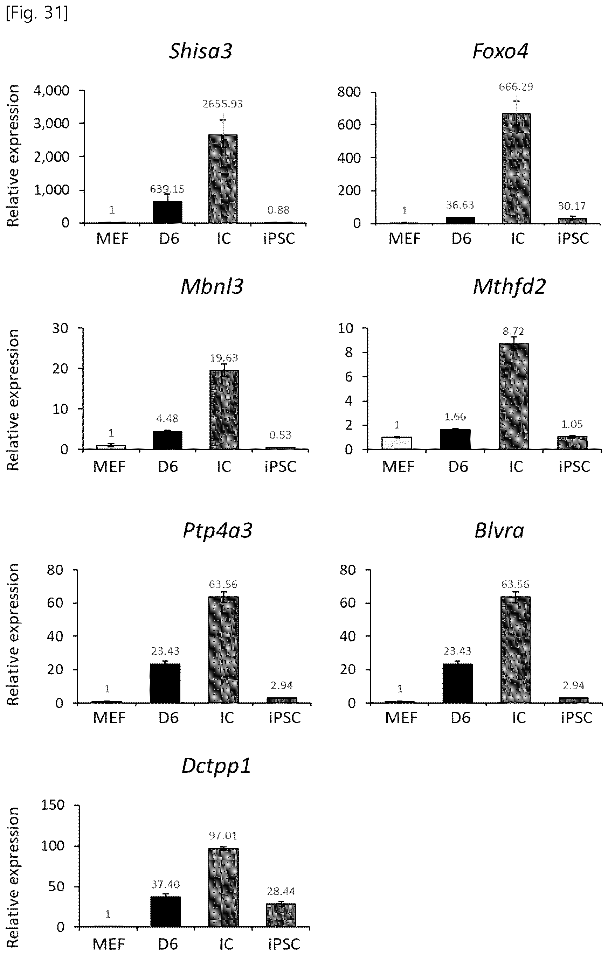

FIG. 31 shows graphs obtained by comparing the expression of the gene markers of intermediate cells (ICs) and induced pluripotent stem cells (iPSCs).

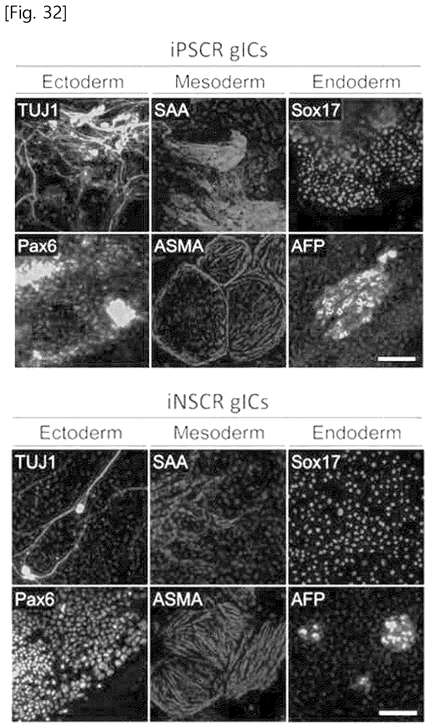

FIG. 32 shows photographs confirming that the cells were naturally differentiated from intermediate cells (ICs) into various cells constituting three germ layers.

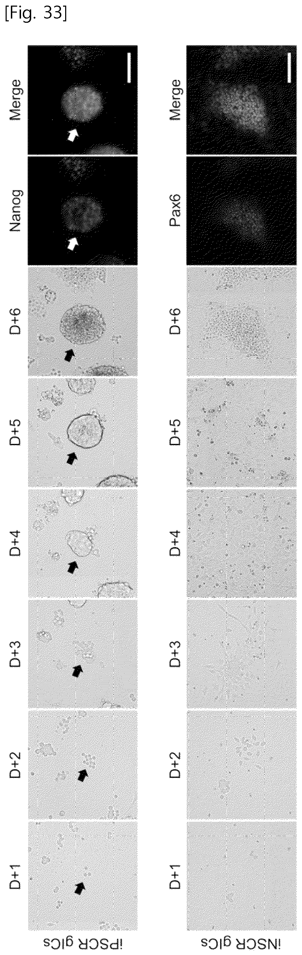

FIG. 33 shows photographs confirming the change from intermediate cells into induced pluripotent stem cells (iPSCs) and neural stem cells (iNSCs).

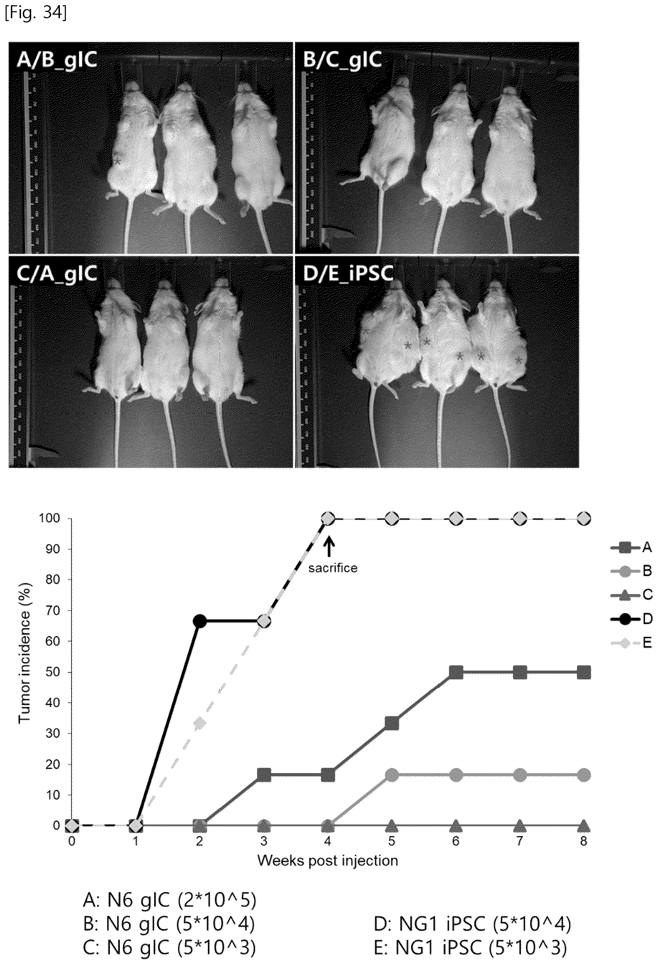

FIG. 34 shows the degree of tumor formation of mice administered intermediate cells and induced pluripotent stem cells (iPSCs).

### Best Mode for Carrying out the Invention

According to an aspect of the present invention, the present invention relates to a method for converting non-pluripotent cells into pluripotent cells, the method including inducing the non-pluripotent cells to express reprogramming factors, thereby overexpressing desmosome-related genes or an epithelial cell differentiation-related gene.

The "pluripotency" refers to the capacity of cells to form all lineages (*i.e*., embryonic compatibility) of the body or somatic cells. The pluripotency may be determined in part by evaluating the pluripotency characteristics of cells. In the present invention, the pluripotency characteristics include, but are not limited to, the ability to differentiate into ectoderm, mesoderm, and endoderm.

The non-pluripotent cells are cells that do not have pluripotency, and refer to cells that have already been differentiated and do not have the ability to differentiate into ectoderm, mesoderm, and endoderm. In an embodiment of the present invention, the non-pluripotent cells may be cells derived from an animal, and more preferably may be cells of a mammal (*e.g*., a human, a mouse, a rat, a cat, a hamster, a guinea pig, a pig, a dog, a horse, a cow, and the like, but is not limited thereto), in particular, human cells, but are not limited thereto. Furthermore, the non-pluripotent cells may be cells harvested from an individual requiring the administration of a customized cell therapy product.

In an embodiment of the present invention, the method of converting non-pluripotent cells into pluripotent cells may be performed *in vivo* or *in vitro.*

In another embodiment of the present invention, the non-pluripotent cells may be cells derived from various tissues such as bone marrow, skin, skeletal muscle, thymus, liver, spleen, heart, brain, eyeball, gastrointestinal tract, cartilage, kidney, testis, ovary, adipose tissue, peripheral blood, and epithelial tissue of a mammal, but are not limited thereto. For example, the non-pluripotent cells may be fibroblasts, organ epithelial cells, prostate epithelial cells, endothelial cells, hepatocytes, B cells, T cells, blood cells, monocytes, cartilage cells, muscle cells, nerve cells, or the like, but are not limited thereto.

The inducing of the cells so that the non-pluripotent cells express the reprogramming factor may enable the non-pluripotent cells to express the reprogramming gene by introducing a gene encoding the reprogramming factor into the non-pluripotent cells or treating the non-pluripotent cells with the reprogramming factor in an exogenous manner.

The term "reprogramming" refers to a method for converting one type of cell into another type of cell. Through reprogramming, this may be converted to cells that are less differentiated than the original cells, or may be converted to specific cells.

The term "reprogramming factor" refers to an agent capable of increasing the effect of cell generation, either alone or in combination with other agents, and includes, but is not limited to, a polynucleotide, a polypeptide, and a small molecule. Exemplary reprogramming factor includes, for example, a transcription factor and a small molecule reprogramming agent. In addition, the reprogramming factor may be one or more proteins selected from among an OCT4 polypeptide (an OCT3 polypeptide or a POU5F1 polypeptide), a SOX2 polypeptide, a NANOG polypeptide, a KLF4 polypeptide, a LIN28 polypeptide, a C-MYC polypeptide, a SV40LT polypeptide, an hTERT polypeptide, a SALL4 polypeptide, a GLIS polypeptide, an ESRRB polypeptide, a DPPA2 polypeptide, an ECAT1 polypeptide, a SOX1 polypeptide, a SOX3 polypeptide, a KLF2 polypeptide, a KLF5 polypeptide, an L-MYC polypeptide, an N-MYC polypeptide, an LRH1 polypeptide, and a UTF1 polypeptide. In particular, the reprogramming factors may be one or more proteins selected from among an OCT4 polypeptide (an OCT3 polypeptide or a POU5F1 polypeptide), a Sox2 polypeptide, a KLF4 polypeptide, and a C-MYC polypeptide, or may be an OCT4 polypeptide, a Sox2 polypeptide, a KLF4 polypeptide, and a C-MYC polypeptide.

Further, the amino acid sequences of the OCT4 polypeptide (OCT3 polypeptide or POU5F1 polypeptide), the SOX2 polypeptide, the NANOG polypeptide, the KLF4 polypeptide, the LIN28 polypeptide, the C-MYC polypeptide, the SV40LT polypeptide, the hTERT polypeptide, the SALL4 polypeptide, the GLIS polypeptide, the ESRRB polypeptide, the DPPA2 polypeptide, the ECAT1 polypeptide, the SOX1 polypeptide, the SOX3 polypeptide, the KLF2 polypeptide, the KLF5 polypeptide, the L-MYC polypeptide, the N-MYC polypeptide, the LRH1 polypeptide, and the UTF1 polypeptide for each species may be obtained from public database (*e.g.*, GenBank or UniProt). As an example, UniProt provides the amino acid sequences of a human OCT4 polypeptide, a human SOX2 polypeptide, a human KLF4 polypeptide, and a human C-MYC polypeptide under the names of UniProtKB - Q01860, UniProtKB - P48431, UniProtKB - O43474, UniProtKB - P01106, and UniProtKB - Q9H9Z2, respectively.

As a method for introducing the gene encoding the reprogramming factor into the cell, a method known in the art may be used, and the gene encoding the reprogramming factor may be introduced into the cell by, for example, transient transfection, microinjection, transduction, electrofusion, calcium phosphate precipitation, liposome-mediated transfection, DEAE Dextran-mediated transfection, polybrain-mediated transfection, electroporation, gene gun, or other known methods for introducing the nucleic acid into the cell, but the method is not limited thereto (Wu et al., J. Bio. Chem., 267:963-967, 1992; Wu and Wu, J. Bio. Chem., 263:14621-14624, 1988).

The reprogramming factor may be provided as RNA, linear DNA, peptide or protein, or a cell extract of pluripotent stem cells.

In addition, when the gene coding the reprogramming factor is introduced into the non-pluripotent cell, the species from which the reprogramming factor and the non-pluripotent cell are derived may be the same. For example, a human reprogramming factor may be introduced into a human cell through transformation.

Furthermore, when the gene coding the reprogramming factor is introduced into the cell, the gene may be introduced using a plasmid vector, a viral vector, or another expression cassette. In addition, a nucleotide sequence encoding the gene coding the reprogramming factor may be inserted into an appropriate vector and introduced into a non-pluripotent cell in order to express a desired polypeptide. Examples of vectors include plasmids, autonomously replicating sequences, and transposable elements. Additional exemplary vectors include, but are not limited to, plasmids, phagemids, cosmids, artificial chromosomes such as yeast artificial chromosome (YAC), bacterial artificial chromosome (BAC) or P1-derived P1-derived artificial chromosome (PAC), bacteriophages such as lambda phages or M13 phages, and animal viruses. Examples of categories of animal viruses useful as vectors include, but are not limited to, retrovirus (including lentivirus), adenovirus, adeno-associated virus, herpesvirus (*e.g*., herpes simplex virus), poxvirus, baculovirus, papillomavirus, and papovavirus (*e.g*., SV40). Examples of expression vectors are pClneo vectors (Promega) for expression in mammalian cells; pLenti4/V5-DEST^{™}, pLenti6/V5-DEST^{™}, and pLenti6.2/V5-GW/lacZ (Invitrogen) for lentivirusmediated gene transfer and expression in mammalian cells.

In another aspect of the present invention, the vector may be an episomal vector that is maintained extrachromosomally. The term "episomal" refers to a vector that is able to replicate without integration into host's chromosomal DNA and without gradual loss from a dividing host cell also meaning that said vector replicates extrachromosomally or episomally. The vector is engineered to harbor the sequence coding for the origin of DNA replication or "ori" from a lymphotrophic herpes virus or a gamma herpes virus, an adenovirus, SV40, a bovine papilloma virus, or a yeast, specifically a replication origin of a lymphotrophic herpes virus or a gamma herpes virus corresponding to oriP of EBV. In a particular aspect, the lymphotrophic herpes virus may be Epstein Barr virus (EBV), Kaposi's sarcoma herpes virus (KSHV), Herpes virus saimiri (HS), or Marek's disease virus (MDV). Epstein Barr virus (EBV) and Kaposi's sarcoma herpes virus (KSHV) are also examples of a gamma herpes virus.

In an embodiment of the present invention, a vector including an expression cassette that causes the expression of reprogramming factors in the presence of an antibiotic such as tetracycline can be introduced into a non-pluripotent cell. In addition, the expression cassette may include a Nanog-GFP reporter. The expression cassette may cause the expression of reprogramming factors in the presence of doxycycline, and in particular, the expression cassette may cause, without limitation, the expression of the OCT4 polypeptide, the Sox2 polypeptide, the KLF4 polypeptide, and the C-MYC polypeptide upon the administration of doxycycline.

A method for confirming whether genes coding reprogramming factors are well introduced in the cells include, for example, molecular biological assays well known to those skilled in the art, such as Southern and Northern blotting, RT-PCR and PCR; biochemical assays, such as detecting the presence or absence of certain proteins/peptides, e.g., by immunological means (*e.g*., ELISAs and Western blots).

In addition, in order to confirm whether genes coding reprogramming factors are well introduced in the cells, the cells into which the reprogramming factor has been introduced can be isolated and can be continuously cultured in a medium including the antibiotics, through the first screening process for the cells having resistance to antibiotics due to the treatment with the antibiotics. The antibiotics may be tetracycline-based antibiotics such as doxycycline, but are not limited thereto.

Further, treating the non-pluripotent cells with the reprogramming factors in an exogenous manner may be performed by bringing the reprogramming factor into direct contact with the cells or culturing the cells in a medium containing the reprogramming factors, but is not limited thereto.

In an embodiment of the present invention, cells induced to express the reprogramming factors may be cultured in a reprogramming medium. As the reprogramming medium, a medium generally used to reprogram into iPSCs, neural stem cells (iNSCs), and dopaminergic neural progenitor cells may be used. The reprogramming medium may or may not include a leukemia inhibitory factor (LIF). For example, the reprogramming medium may be a medium including KnockOut DMEM (Thermo Fisher Scientific), KnockOut Serum Replacement (Thermo Fisher Scientific), FBS, Glutamax, MEM-NEAA, 1X Penicillin/Streptomycin, and β-mercaptoethanol (Thermo Fisher Scientific), a medium including KnockOut DMEM (Thermo Fisher Scientific), KnockOut Serum Replacement (Thermo Fisher Scientific), FBS, Glutamax, MEM-NEAA, 1X Penicillin/Streptomycin, β-mercaptoethanol (Thermo Fisher Scientific) and LIF (Millipore, Billerica, MA, USA), a medium including advanced DMEM/F12 (Thermo Fisher Scientific), neurobasal media (Thermo Fisher Scientific), BSA, N2 (Thermo Fisher Scientific), B27 (Thermo Fisher Scientific), Glutamax, β-mercaptoethanol, FGF2 (Peprotech, Rocky Hill, NJ, USA), FGF4 (Peprotech) and EGF (Peprotech), a medium including advanced DMEM/F12 (Thermo Fisher Scientific), neurobasal media (Thermo Fisher Scientific), BSA, N2, B27, Glutamax, FGF8b (Peprotech), SHH (Peprotech), and β-mercaptoethanol, or the mTeSR 1 (STEMCELL Technologies, Vancouver, Canada) medium supplemented with CHIR99021 (Tocris Bioscience, Bristol, UK), A83 01 (Tocris), and NaB (Sigma Aldrich); a medium including advanced DMEM/F12 (Thermo Fisher Scientific), neurobasal media (Thermo Fisher Scientific), BSA, N2 (Thermo Fisher Scientific), B27 (Thermo Fisher Scientific), Glutamax, β-mercaptoethanol, FGF2 (Peprotech, Rocky Hill, NJ, USA), FGF4 (Peprotech), EGF (Peprotech), CHIR99021, A83 01, and hLIF (Peprotech).

In addition, in an embodiment of the present invention, the cells induced to express the reprogramming factors may overexpress desmosome-related genes or epithelial cell differentiation-related genes.

The desmosome is an intercellular junction of the epithelium, which is a kind of epithelial tissue, and is an intercellular junction connecting keratin filaments of adj acent cells. The desmosome is composed of desmosomal cadherin (*i.e*., desmocollin (Dsc) and desmoglein (Dsg)), armadillo protein (*i.e*., plakoglobin and plakophilins), and desmoplakin (Dsp) which is a cytoskeletal linker protein.

Meanwhile, the cadherin protein and the desmocollin protein are involved in cellcell adhesion, and the desmoplakin (Dsp) protein performs a function of attaching the desmosome to keratin.

*Dsg1, Dsg2, Dsg3, Dsg4, Dsc1, Dsc2, Dsc3, plakoglobin (Jup), plakophilin 1* to *plakophilin 3 (Pkp1* to *Pkp3*), *Pe*rp, *D*sp, *krt8,* and the like are known as the desmosome-related genes or the epithelial cell differentiation-related genes. However, in the present invention, desmosome-related genes or epithelial cell differentiation-related genes, of which the expression level is measured, is not limited thereto. Meanwhile, the desmosome-related genes or the epithelial cell differentiation-related genes of which the expression level is measured in the method for converting non-pluripotent cells into pluripotent cells may be at least one gene selected from among *Dsp, Evpl, Dsg1a, Dsg1b, Dsg1c, Dsg3, Jup, Perp, Pkp1* and *Krt8,* or at least one gene selected from among *Dsg3, Dsg4, Dsp, Evpl, Jup, Perp,* and *Pkp1,* and the desmosome-related genes or the epithelial cell differentiation-related genes may be *Dsp* and *Pkp1.* The nucleotide sequence of the desmosome-related genes or the epithelial cell differentiation-related genes may be obtained from a known database such as GenBank, *e.g*., GenBank is GenBank Accession ID: 109620 or GenBank Accession ID: 832, which provides a nucleotide sequence of *Dsp* gene.

In another embodiment of the present invention, the expression level of the gene may be measured at the nucleic acid level of the protein coded by the gene or the mRNA or gene coded by the gene. The amount of protein may be determined using an antibody specifically binding to the protein coded by the gene, and preferably, the level of protein expression itself may be measured without using the antibody.

For example, the protein expression measurement may be performed using protein chip analysis, immunoassay, ligand binding assay, matrix desorption/ionization time of flight mass spectrometry (MALDI-TOF) analysis, surface enhanced laser desorption/flight mass spectrometry (SELDI-TOF) analysis, radioimmunoassay, radial immunodiffusion, radioimmunodiffusion, Ouchterlony immunodiffusion, Rocket immunoelectrophoresis, immunohistochemistry analysis, immunocytochemistry analysis, complement fixation analysis, 2D electrophoresis analysis, liquid chromatography-mass spectrometry (LC-MS), liquid chromatography-mass spectrometry/mass spectrometry (LC-MS/MS), Western blot, enzyme linked immunosorbent assay (ELISA), and the like, but is not limited thereto.

In addition, the mRNA expression measurement of the gene may be performed using transcriptional polymerase reaction (RT-PCR), competitive reverse transcription polymerase reaction (competitive RT-PCR), real-time reverse transcription polymerase reaction (real-time RT-PCR), RNase protection assay (RPA), Northern blotting, single cell RNA sequencing, DNA chip or the like, but is not limited thereto. The agent for measuring the mRNA level of the gene is preferably primer pairs or probes, and since the nucleic acid information of the genes is known to GenBank or the like, a person skilled in the art may design primers or probes that specifically amplify a specific region of these genes based on the sequence. In addition, the agent for measuring the mRNA level of the gene may include a primer pair, probe, or antisense nucleotide that specifically binds to the gene.

In an aspect of the present invention, the present invention may further include overexpressing and then reducing the desmosome-related genes or the epithelial cell differentiation-related genes. In this case, the desmosome-related genes or the epithelial cell differentiation-related genes may be reduced to a lower level compared to the expression of the corresponding gene in induced pluripotent stem cells.

In addition, in an embodiment of the present invention, the method for converting non-pluripotent cells into pluripotent cells may further include separating cells in which desmosome-related genes or epithelial cell differentiation-related genes are overexpressed. The cell separation may be performed using a method for separating cells having a high expression level of a target protein or a target gene known in the art. For example, cells may be separated by centrifugation using a difference in weight or density of cells, cell separation using a flow cytometer, or the like, may be mechanically separated or separated by treatment with enzymes, or may be separated using the form of cells.

In another embodiment of the present invention, the method for converting non-pluripotent cells into pluripotent cells may include separating cells at a period when desmosome-related genes or epithelial cell differentiation-related genes decrease sharply.

Reprogramming may be restricted before, after, or during the cell separation by methods known in the art. The restriction of reprogramming may mean stopping some or all of reprogramming. For example, the reprogramming medium may be changed by adding a component that interferes with the generation of induced pluripotent stem cells (*e.g*., JAK inhibitor) to the reprogramming medium or omitting feeder *MEF.* A material known in the art may be used as a material that restricts the reprogramming, for example, JMJD3 may be used, but is not limited thereto. In addition, the method for converting non-pluripotent cells into pluripotent cells may include restricting the expression of reprogramming factors. The restricting of the expression of the reprogramming factor may be restricting or stopping the expression of some or all of the reprogramming factors.

Conditions for restricting the expression of the reprogramming factor may include changing the condition for expressing the reprogramming factor (*e.g*., a condition for excluding tetracycline from the medium in which the cells are cultured, when the cells are allowed to express the reprogramming factor during the administration of tetracycline, such as doxycycline), or treating the cells with a viral vector for knocking-out the reprogramming factor, but are not limited thereto. In addition, the cell separation in the method for converting non-pluripotent cells into pluripotent cells may be performed while inhibiting the expression of at least one gene selected from the group consisting of *Nanog, Rex1,* and *Esrrb.* In another aspect of the present invention, the method for converting non-pluripotent cells into pluripotent cells may further include selecting cells that further express *Shisa8* gene among cells in which the expression level of the desmosome-related genes or the epithelial cell differentiation-related genes is higher than the expression level of the corresponding gene in iPSCs. The *Shisa8* gene is a gene encoding the Shisa family member 8 protein. The GenBank provides the nucleotide sequence of the *Shisa8* gene, *e.g.,* the GenBank accession ID of the human *Shisa8* gene is 440829. In addition, the expression of the *Shisa8* gene may be measured by the above-described method for measuring the expression level of the gene. In another aspect of the present invention, the method for converting non-pluripotent cells into pluripotent cells may further include culturing the selected cells in a reprogramming medium. The selected cells may have a feature in which re-desorption is possible. In addition, the method for converting non-pluripotent cells into pluripotent cells may further include culturing the selected cells in a reprogramming medium. The reprogramming medium may generally be a medium used to reprogram into iPSCs, neural stem cells (iNSCs), and dopaminergic neural progenitor cells as described above. Meanwhile, as the reprogramming medium, a medium suitable for reprogramming into ectoderm, endoderm, or mesoderm cells, which is known in the art, may be used.

According to an aspect of the present invention, the method for converting non-pluripotent cells into pluripotent cells may further include separating cells of which the expression level of the *Spink2* gene is higher than that of induced pluripotent cells (iPSCs). The *Spink2* gene is a gene encoding serine protease inhibitor Kazal-type 2 (Spink2), also known as an acrosin-trypsin inhibitor. The *Spink2* gene is a gene marker that is specifically expressed in the pluripotent cells of the present invention, which is not substantially expressed in conventional pluripotent stem cells.

In addition, the GenBank provides the nucleotide sequence of the *Spink2* gene, *e.g.,* the GenBank accession ID of the human *Spink2* gene is 6691. In addition, the expression of the *Spink2* gene may be measured by the above-described method for measuring the expression level of the gene.

In an embodiment of the present invention, the pluripotent cells may exhibit increased expression of the *Spink2* gene by 1-700 times (exclusive of 1) compared to the induced pluripotent stem cells. Specifically, the *Spink2* gene of the pluripotent cells may exhibit the expression increased by 10 times or more, 1-700 times (exclusive of 1), 3-650 times, 10-600 times, or 20-570 times compared to the induced pluripotent stem cells. In an embodiment of the present invention, the *Spink2* gene of the pluripotent cells exhibited an expression level of about 569 times (39.81/0.07 = 568.71) higher than that of induced pluripotent stem cells.

In another embodiment of the present invention, the expression level of the desmosome-related genes or the epithelial cell differentiation-related gene sof the pluripotent cells may be at least about 2 times or more, at least 3 times, at least 5 times, at least 10 times, at least 15 times, or at least 20 times lower than that of the *Spink2* gene. In addition, the expression level of the desmosome-related genes or the epithelial cell differentiation-related genes may be about 2-20 times, about 5-15 times, or about 7-10 times lower than that of the *Spink2* gene.

In another aspect of the invention, the pluripotent cells may exhibit reduced expression of at least one gene selected from the group consisting of *Nanog, Rex1,* and *Esrrb* compared to the expression level of the corresponding genes in the iPSCs. The GenBank provides the nucleotide sequences of *Nanog, Rex1* and *Esrrb,* and the expression of *Nanog, Rex1* and *Esrrb* genes may be determined by the above-described method for measuring the expression level of the gene.

In addition, the pluripotent cell may exhibit decreased expression of at least one gene selected from the group consisting of *Nanog, Rexl* and *Esrrb* by 10 times or more, 11 times or more, 12 times or more, 13 times or more, 14 times or more, 15 times or more, 16 times or more, 17 times or more, 18 times or more, 19 times or more, 20 times or more, or 100 times or more compared to the expression level of the corresponding genes in the iPSCs. Furthermore, the expression of the *Nanog* gene may be reduced by 100 times or more compared to the expression level in the iPSCs.

In an aspect of the present invention, the pluripotent cells may be cells in which tumorigenicity is reduced compared to the iPSCs.

In another aspect of the present invention, the pluripotent cells may be differentiated into cells constituting ectoderm, mesoderm, or endoderm.

In another aspect of the present invention, the pluripotent cells may be formed by reducing the expression of the desmosome-related genes or epithelial cell differentiation-related genes in cells in which these genes are overexpressed. In addition, the pluripotent cells may be cells formed by reducing the expression of the *Dsp* gene in cells expressing the *Dsp* gene and the *Shisa8* gene.

In another aspect of the invention, the pluripotent cells may be cells that express the *Spink2* gene and exhibit increased expression of at least one gene selected from the group consisting of *Shisa3, Foxo4, Ptp4a3, Blvra, Mbnl3, Mthfd2,* and *Dctpp1* and the *Spink2* gene as compared to the expression level of the corresponding genes in the iPSCs. The GenBank provides the nucleotide sequences of *Shisa3, Foxo4, Ptp4a3, Blvra, Mbnl3, Mthfd2,* and *Dctpp1,* and a method for measuring the expression of *Shisa3, Foxo4, Ptp4a3, Blvra, Mbnl3, Mthfd2,* and *Dctpp1* genes are the same as the above-described methods used in measuring the expression level of the gene.

The *Shisa3* gene is a gene encoding the single transmembrane protein Shisa3. The Shisa3 and the *Shisa3* gene encoding the same may have a sequence published in Gene ID: 330096 (NCBI Reference Sequence: NC_000071.7) or Gene ID: 152573 (NCBI Reference Sequence: NC_000004.12). The Shisa3 is known to play an essential role in the maturation of prostate mesenchymal cells by independently attenuating the signaling system of each of FGF and WNT.

The *Foxo4* gene is a gene encoding forkhead box protein O4 peptide (Foxo4). The Foxo4 and the *Foxo4* gene encoding the same may have a sequence published in Gene ID: 54601 (NCBI Reference Sequence: NC_000086.8) or Gene ID: 4303 (NCBI Reference Sequence: NC_000023.11). The Foxo4 is a transcription factor included in regulating the insulin signaling system and is known to be involved in inhibiting a cell cycle or increasing proteasome activity in embryonic stem cells.

The *Ptp4a3* gene is a gene encoding protein tyrosine phosphatase type IVA 3 (Ptp4a3). The Ptp4a3 and the *Ptp4a3* gene encoding the same may have a sequence published in Gene ID: 19245 (NCBI Reference Sequence: NC_000081.7) or Gene ID: 4303 (NCBI Reference Sequence: NC_000023.11). The Ptp4a3 belongs to a protein tyrosine phosphatase (PTP) group, and the PTP is a cell signaling material that serves to regulate various cell processes. The Ptp4a3 is known to be involved in enhancing cell proliferation and mobility or promoting tumor metastasis.

The *Blvra* gene is a gene encoding biliverdin reductase A (Blvra). The Blvra and the *Blvra* gene encoding the same may have a sequence published in Gene ID: 109778 (NCBI Reference Sequence: NC_000068.8) or Gene ID: 644 (NCBI Reference Sequence: NC_000007.14). The Blvra belongs to the biliverdin reductase family which catalyzes the conversion from biliverdin to bilirubin in the presence of NADPH or NADH. It is known that mutations in the *Blvra* gene is identified in a disease such as hyperbiliverdinemia or cholestasis.

The *Mbnl3* gene is a gene encoding muscleblind-like protein 3 (Mbnl3). The *Mbnl3* gene may have a sequence published in Gene ID: 171170 (NCBI Reference Sequence: NC_000086.8) or Gene ID: 55796 (NCBI Reference Sequence: NC_000023.11). The Mbnl3 is known to be involved in the regulation of alternative splicing and to play a role in the pathology of muscular atrophy.

The *Mthfd2* gene is a gene encoding Mthfd2. The Mthfd2 and the *Mthfd2* gene encoding the same may have a sequence published in Gene ID: 17768 (NCBI Reference Sequence: NC_000072.7) or Gene ID: 10797 (NCBI Reference Sequence: NC_000002.12). The Mthfd2 functions as a homodimer and forms a complex with magnesium or inorganic phosphate, thereby enabling to bind to NAD.

The *Dctpp1* gene is a gene encoding dCTP pyrophosphatase 1 (Dctpp1). The Dctpp1 and the *Dctpp1* gene encoding the same may have a sequence published in Gene ID: 66422 (NCBI Reference Sequence: NC_000073.7) or Gene ID: 79077 (NCBI Reference Sequence: NC_000016.10). The Dctpp1 is an enzyme that converts dCTP into dCMP and inorganic pyrophosphate. Diseases associated with Dctpp1 include aspartylglucosaminuria and otosclerosis.

The pluripotent cells may have a granular morphology, but are not limited thereto. In addition, the pluripotent cells may be differentiated into CD34 cells, hematopoietic endothelial cells, hematopoietic stem and progenitor cells (HSCs), hematopoietic pluripotent progenitor cells, T cell precursors, NK cell precursors, T cells, NKT cells, NK cells, and B cells, but are not limited thereto. Furthermore, the pluripotent cells may be differentiated into iPSCs, iNSCs, or iDPs.

In one aspect of the present invention, the present invention relates to pluripotent cells prepared by the method for converting cells non-pluripotent cells into pluripotent cells or cells having increased expression of desmosome-related genes or epithelial cell differentiation-related genes. The cells having increased expression of desmosome-related genes or epithelial cell differentiation-related genes may be cells expressing the *Dsp* gene and the *Shisa8* gene. The cells expressing the *Dsp* gene and the *Shisa8* gene produced through the cells having increased expression of desmosome-related genes or epithelial cell differentiation-related genes may be cells expressing the *Spink2* gene and additionally expressing one or more marker genes selected from the group consisting of *Shisa3, Foxo4, Ptp4a3, Blvra, Mbnl3, Mthfd2,* and *Dctpp1,* and may be granular cells, but the present invention is not limited thereto. The characteristics of the pluripotent cells are the same as described above.

The cells expressing the *Dsp* gene and the *Shisa8* gene formed in the process of converting the non-pluripotent cells into pluripotent cells are further cultured in the iPSC reprogramming medium, the iNSC reprogramming medium, the iDP reprogramming medium, or a medium known to be suitable for reprogramming of cells of interest such as the triploblastic (ectoderm, mesoderm, and endoderm) cells used in Examples of the present specification, or are differentiated into the cells of interest or naturally dedifferentiated into three germ layers when providing conditions suitable for reprogramming into the cells of interest. As the medium known to be suitable for reprogramming the cells of interest and the conditions suitable for reprogramming the cells of interest, the medium and conditions known in the art to which the present invention pertains may be used.

The endoderm, mesoderm, and ectoderm form three germ layers, and in the endoderm, cells forming the stomach, colon, liver, pancreas, urinary bladder, the lining of urethra, epithelial portion of the airways, lung, pharynx, thyroid, parathyroid, and small intestine tissues are generated, in the mesoderm, cells forming skeletal muscle, bone, dermis, connective tissue, urinary reproductive system, heart, blood (lymphocytes), kidney and spleen are generated, and in the ectoderm, cells forming the central nervous system, lens of the eye, the skull and sensation, ganglion and nerve, pigment cells, head connective tissues, epidermis, hair, and mammary glands are generated.

In another aspect of the present invention, the present invention relates to cells in which the expression of desmosome-related genes or epithelial cell differentiation-related genes is higher than the expression level of the corresponding gene in the iPSCs, or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding gene in the iPSCs. The cells in which the expression of desmosome-related genes or epithelial cell differentiation-related genes is higher than the expression level of the corresponding gene in the iPSCs may be cells expressing at least one gene selected from the group consisting of *Dsp, Evpl, Dsg1a, Dsglb, Dsglc, Dsg3, Jup, Perp, Pkpl,* and *Krt8,* or at least one gene selected from the group consisting of *Dsg3, Dsg4, Dsp, Evpl, Jup, Perp,* and *Pkpl,* and in particular, may be cells expressing the *Dsp* gene and *Shisa8* gene, but the present invention is not limited thereto. In addition, the cells in which the expression level of *Spink2* gene is higher than the expression level of the gene in the iPSCs may be cells exhibiting pluripotency that is the potential to differentiate into any one of ectodermal cells, mesodermal cells, or endodermal cells, and may have the characteristics of the above-described pluripotent cells.

In another aspect of the present invention, the present invention relates to a method for producing cells, the method including inducing the differentiation of the pluripotent cells; or cells in which the expression of desmosome-related genes or epithelial cell differentiation-related genes is higher than the expression level of the corresponding gene in the iPSCs, or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding gene in the iPSCs. The inducing of the cell differentiation may be performed by culturing cells of interest by applying conditions known to be suitable for the differentiation of the cells of interest, such as ectodermal cells, mesodermal cells, endodermal cells, iPSCs, or neural stem cells (iNSCs), or by creating such conditions *in vivo* or *ex vivo.* In another aspect of the present invention, the present invention relates to a composition for cell transplantation or biological tissue regeneration, the composition including, as active ingredients: the pluripotent cells; cells in which the expression of desmosome-related genes or epithelial cell differentiation-related genes is higher than the expression level of the corresponding gene in the iPSCs, or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding gene in the iPSCs; or the differentiation-induced cells. The biological tissue may be a damaged tissue selected from among tissue with ulcer or bedsore, brain tissue damaged by cell degeneration, brain tissue partially lost by surgical operation, brain tissue damaged by traumatic brain disease, brain tissue damaged by inflammatory brain disease, damaged bone tissue, damaged periodontal tissue, tissue damaged by central nervous system disease, and tissue damaged by intractable dermatitis, and the biological tissue regeneration may be restoration of the damaged tissue, epidermis regeneration, regeneration of secretory glands or hair follicles, microangiogenesis of dermal tissue, wound healing, correction of flaws of soft tissue, bone healing or bone regeneration, cartilage regeneration, or the like, but is not limited thereto.

In an aspect of the present invention, the present invention relates to a method for converting non-pluripotent cells into the pluripotent cells, iPSCs or iNSCs, the method including overexpressing desmosome-related genes or an epithelial cell differentiation-related genes in the non-pluripotent cells, wherein the method can increase the efficiency of reprogramming the non-pluripotent cells into the pluripotent cells, iPSCs or iNSCs. The increase in reprogramming efficiency means that the number of obtained pluripotent cells, iPSCs or iNSCs is increased to more than 1-fold or the time required to obtain pluripotent cells, iPSCs or iNSCs is shortened, compared to the case of converting non-pluripotent cells to the pluripotent cell, iPSCs or iNSCs without the overexpressing of desmosome-related genes or epithelial cell differentiation-related genes in the non-pluripotent cells.

Meanwhile, in an embodiment of the present invention, the overexpressing of desmosome-related genes or epithelial cell differentiation-related genes in the method for converting the non-pluripotent cells into the pluripotent cells, iPSCs, or iNSCs may be performed using the above-described method used for introducing a gene encoding the reprogramming factor into the non-pluripotent cell. In addition, the characteristics of the non-pluripotent cells and the pluripotent cells are the same as described above. The overexpressed desmosome-related genes or epithelial cell differentiation-related genes may be at least one gene selected from the group consisting of *Dsp, Evpl, Dsg1a, Dsg1b, Dsg1c, Dsg3, Jup, Perp, Pkp1,* and *Krt8,* or may be preferably *Dsp,* but is not limited thereto. In addition, the method for converting the non-pluripotent cells into the pluripotent cells, iPSCs, or iNSCs may be an iPSC reprogramming method, a cross-differentiation method, a direct cross-differentiation method, or a direct pre-differentiation factor-mediated reprogramming method, or a method for converting the fate of cells known in the art to which the present invention belongs, but is not limited thereto. In an aspect of the present invention, the present invention relates to a method for evaluating the efficacy or toxicity of a substance to be tested, the method including bringing: the pluripotent cells; cells in which the expression of desmosome-related genes or epithelial cell differentiation-related genes is higher than the expression level of the corresponding gene in induced pluripotent stem cells (iPSCs), or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding gene in the iPSCs; or the differentiation-induced cells, into contact with the substance to be tested.

In an aspect of the present invention, isolated cells in the method for converting the non-pluripotent cells into the pluripotent cells of the present invention, ectodermal cells, mesodermal cells, endodermal cells, iPSCs, iNSCs, iDPs, or the like, which are produced by differentiating the isolated cells, may be used in the field of regenerative medicine. For example, in order to prepare a patient-specific cell therapy product, isolated cells obtained by converting the fate of somatic cells obtained from an individual by the method for converting the non-pluripotent cells into the pluripotent cells, ectodermal cells, mesodermal cells, endodermal cells, iPSCs, iNSCs, iDPs, or the like, which are produced by differentiating the isolated cells, may be transplanted or administered to the individual.

In addition, in an embodiment of the present invention, the cells may be transplanted in an amount sufficient to cause measurable amelioration of a marker of treatment symptoms or conditions upon the transplantation of the cells into a patient. In order to transplant an amount of cells effective to achieve a desired therapeutic response to a particular subject, the actual transplant level of the cells of the therapeutic composition may be altered. The selected transplant level may depend on a variety of factors including, but not limited to, the therapeutic composition, formulation, the route of administration, combination with other drugs or treatments, severity of the condition being treated, the physical condition of the subject, prior medical history of the subject being treated and the experience and judgment of the clinician or practitioner administering the therapy. In general, the amounts and methods of transplant scheduled should be sufficient to result in slowing, and preferably inhibiting progression of the condition, and also preferably causing a decrease in one or more symptoms or markers of the condition.

In another aspect of the present invention, the present invention relates to a method for regenerating biological tissue by administering, to an individual, the pluripotent cells; cells in which the expression of desmosome-related genes or epithelial cell differentiation-related genes is higher than the expression level of the corresponding gene in induced pluripotent stem cells (iPSCs), or the expression level of *Spink2* gene is higher than the expression level of the gene in the iPSCs, and the expression level of at least one gene selected from the group consisting of *Nanog, Rex1,* and *Esrrb* is reduced compared to the expression level of the corresponding genes in the iPSCs; or the differentiation-induced cells. The cells may be administered in a manner that allows the cells to graft to a site in need of regeneration of biological tissue and reconstitute or regenerate a functionally deficient area. For example, the cells may be directly transplanted into the parenchyma or intrathecal sites of the central nervous system, depending on the disease being treated. In some embodiments, the cells may be injected into the patient by intravenous, intravertebral, intracerebroventicular, intrathecal, intraarterial, intramuscular, intraperitoneal, subcutaneous, intraocular, retrobulbar space and combinations thereof, or injected or deposited in the form of bolus injection or continuous infusion, but the method is not limited thereto. In addition, the cells may be directly transplanted to a site in need of tissue regeneration. In addition, the regenerated biological tissue is the same as described above with respect to the composition for cell transplantation or biological tissue regeneration.

As used herein, the term "individual" refers to any animal, preferably, a human patient, livestock, or an another animal raised in a house.

In another aspect of the present invention, the present invention relates to a use of cells for use in the preparation of a medicament for treating a disease in an individual or in the preparation of a composition for cell transplantation or biological tissue regeneration, wherein the cells are isolated cells in the method for converting non-pluripotent cells into pluripotent cells, ectodermal cells, mesodermal cells, endodermal cells, iPSCs, iNSCs, or iDPs, and the like which are produced by differentiating the isolated cells.

### Modes for the Invention

Hereinafter, the present invention will be described in more detail with reference to the following examples. The scope of the present invention is not limited thereto.

Meanwhile, the types of primers and the types of CRISPR sgRNA used in the following examples are summarized in Tables 1 and 2, respectively, and the types of antibodies used in immunofluorescence staining and Western blot are shown in Table 3.

**[Table 1]**

| Mouse | | | | |
|---|---|---|---|---|
| **Gene** | **Forward primer** | **SEQ ID NO** | **Reverse primer** | **SEQ ID NO** |
| *Nanog* | CCTCCAGCAGATGCAAGAACT | 1 | CTTCAACCACTGGTTTTTCTG | 2 |
| *Rex1* | GGCTGCGAGAAGAGCTTTATT | 3 | AGCATTTCTTCCCGGCCTTT | 4 |
| *Esrrb* | AGGCTCTCATTTGGGCCTAGC | 5 | ATCCTTGCCTGCCACCTGTT | 6 |
| *Pax6* | CGCGGATCTGTGTTGCTCAT | 7 | CTTAAATCCATGGCAAATCTT | 8 |
| *Sox1* | GGCCGAGTGGAAGGTCATGT | 9 | TCCGGGTGTTCCTTCATGTG | 10 |
| *Map2* | GCCAGCCTCGGAACAAACA | 11 | GCTCAGCGAATGAGGAAGGA | 12 |
| *Dcx* | CCATGTGTGTGAGGTGTTGTG | 13 | TCGATGGAGAACAGAGCTTGA | 14 |
| *Dsp* | AATGAGGAGCTTAGCGAAATCC | 15 | CCTTCAGTTGCAGATAGGTGAG | 16 |
| *Evpl* | AACCAGGCTTTACAACACCAG | 17 | AGGCGGAAGTACCATCTTCTC | 18 |
| *Dsc1* | GGTCAAGGAATCAAAACACAGC | 19 | CCAAGCCGAGGTTGAGTGAAA | 20 |
| *Dsc2* | ATGGCGGCTGTGGGATCTAT | 21 | GCAAGGATCGCAAGGGTCAA | 22 |
| *Dsc3* | AGTTTGAAAGAGTGTCTCAGCTC | 23 | ACAACAGCTCTGGTCGGATAA | 24 |
| *Dsg1a* | ACTGGCTGGGTAACTGGTTC | 25 | AGGGTTAGTGTTAGGGCGAGT | 26 |
| *Dsg1b* | GCAGTGGTGGTAATCGTGACC | 27 | GGATTTTGCCTACCGGGAGTG | 28 |
| *Dsg1c* | CATCCACTGAGAAACCTGTGAC | 29 | CTGTGGCCCTTCTACAGCC | 30 |
| *Dsg2* | CGTGGTTGAAGGCATTCATTTC | 31 | TAGCTGCTTGACCAGTGTCTT | 32 |
| *Dsg3* | GCCACGGATGCAGATGAAC | 33 | CGGACTTCTCCGGTGTTTC | 34 |
| *Dsg4* | GGTGGTAAAGTTAAGTGCCACA | 35 | CTTCTCCAGTGTACCTGTTCAC | 36 |
| *Pkp1* | AACCACTCTCCGCTCAAGAC | 37 | CTTCTGCCGTTTGACGGTCAT | 38 |
| *Jup* | TGGCAACAGACATACACCTACG | 39 | GGTGGTAGTCTTCTTGAGTGTG | 40 |
| *Krt8* | TCCATCAGGGTGACTCAGAAA | 41 | CCAGCTTCAAGGGGCTCAA | 42 |
| *Krt16* | TGGCGAGAATATCCACTCCTC | 43 | TGAAGCTGGTTGAACCTTGCT | 44 |
| *Krt17* | ACCATCCGCCAGTTTACCTC | 45 | ACTACCCAGGCCACTAGCTG | 46 |
| *Shisa8* | CTCTACTCGTGCTGGTCGGCAT | 47 | ACCTGGCTGCTTGAGAAGTTCTG | 48 |
| *Cdk1* | AGAAGGTACTTACGGTGTGGT | 49 | GAGAGATTTCCCGAATTGCAGT | 50 |
| *Rpl7* | ACCGCACTGAGATTCGGATG | 51 | GAACCTTACGAACCTTTGGGC | 52 |
| *Shisa3* | ACCTGTTTGAGACCCAAGGAG | 53 | CAGTGGTGTAAGGTGGGGG | 54 |
| *Foxo4* | CTTCCTCGACCAGACCTCG | 55 | ACAGGATCGGTTCGGAGTGT | 56 |
| *Ptp4a3* | CATCACTGTTGTGGACTGGC | 57 | TGGATGGCGTCCTCGTACTT | 58 |
| *Blvra* | CCTGATTGGATATGTGTCCAGAC | 59 | CGACATCAACCTCTTGGCTG | 60 |
| *Mbnl3* | TTGATTCACTAAAGGGTCGGTG | 61 | CTGCGGCAGTCTTCTGTTGA | 62 |
| *Mthfd2* | AGTGCGAAATGAAGCCGTTG | 63 | GACTGGCGGGATTGTCACC | 64 |
| *Dctpp1* | CGCGGTACTGTGGGACAAG | 65 | CAGGCTCTGTATCAGACTTCCA | 66 |

| Human | | | | |
|---|---|---|---|---|
| **Gene** | **Forward primer** | **SEQ ID NO** | **Reverse primer** | **SEQ ID NO** |
| *DSP* | GCAGGATGTACTATTCTCGGC | 67 | CCTGGATGGTGTTCTGGTTCT | 68 |
| *RPL7* | CCAAATTGGCGTTTGTCAG | 69 | GCATGTTCGAAGCCTTGTTG | 70 |

| Zebrafish | | | | |
|---|---|---|---|---|
| **Gene** | **Forward primer** | **SEQ ID NO** | **Reverse primer** | **SEQ ID NO** |
| *dspa* | GACCTGCTTCTGAGGTGGAC | 71 | CTCCTGGCTTCTCTGGATTG | 72 |
| *dspb* | GTCAGGGCTCAGAGCTGTTC | 73 | TGAAGACGCGGTTTCTTTCT | 74 |
| *msx1b* | CACCTTACGCAAGCACAAGA | 75 | GTGAGGTTGAGGGAGTTGGA | 76 |
| *fgf20a* | TCTCAGAGCAGAGGCAGTCA | 77 | TCTCTCGACCTTCCAGGCTA | 78 |
| *her6* | GGTTAACACCGAGGTCAGGA | 79 | TGAACCATGGGTTGACTGAA | 80 |
| *hspd1* | GTGTTGACCTGCTGGCTGAT | 81 | ACCAGCTTGGCTCCGATATT | 82 |
| *rpl13a* | TCTGGAGGACTGTAAGAGGT | 83 | AGACGCACAATCTTGAGAGC | 84 |

**[Table 2]**

| **Name** | **Oligo sequence (5'-3')** |
|---|---|
| dspa sgRNA-1 | taatacgactcactataGGGGAGCCTGGTCTGAGAACgttttagagctagaa |
| dspa sgRNA-2 | taatacgactcactataGGGCCAGACCTCACCGCCGTgttttagagctagaa |
| dspa sgRNA-3 | taatacgactcactataGGGTAAACTGTAGCCGACGGgttttagagctagaa |
| dspb sgRNA-1 | taatacgactcactataGGGTTCAAGAGGCACAGTCAgttttagagctagaa |
| dspb sgRNA-2 | taatacgactcactataGGAAGTGCATCTCCAGACTGgttttagagctagaa |
| dspb sgRNA-3 | taatacgactcactataGGTGCTGTGCTCCTGCAAACgttttagagctagaa |
| Scrambled sgRNA | taatacgactcactataGCAGGCAAAGAATCCCTGCCgttttagagctagaa |
| sgRNA tail (the sgRNA invariable 3' end) | AAAAGCACCGACTCGGTGCCACTTTTTCAAGTTGATAACGGA CTAGCCTTATTTTAACTTGCTATTTCTAGCTCTAAAAC |

**[Table 3]**

| **Antibody used for immunofluorescence staining (*: secondary antibody)** | **Supplier** | **Product No** | **Dilution ratio** |
|---|---|---|---|
| Rabbit anti-Nanog | Abcam | ab80892 | 1:500 |
| Rabbit anti-Oct4 | Santa Cruz Biotechnology | SC-9081 | 1:250 |
| Mouse anti-SSEA-1 | R&D systems | MAB2153 | 1:250 |
| Mouse anti-SSEA-4 | R&D systems | MAB4304 | 1:100 |
| Rabbit anti-Pax6 | BioLegend | PRB-278p-100 | 1:500 |
| Rabbit anti-Nestin | BioLegend | PRB-315C | 1:250 |
| Goat anti-FoxA2 | Santa Cruz Biotechnology | SC-6554 | 1:500 |
| Goat anti-Gfap | Dako | Z0334 | 1:500 |
| Mouse anti-Tuj1 | BioLegend | 802001 | 1:1000 |
| Mouse anti-SAA | Abcam | ab9465 | 1:200 |
| Mouse anti-ASMA | R&D systems | MAB1420 | 1:1000 |
| Goat anti-Sox17 | R&D systems | MAB1924 | 1:250 |
| Rabbit anti-AFP | Dako | A0008 | 1:250 |
| Rabbit anti-Dsc3 | Abcam | ab126768 | 1:15000 |
| Mouse anti-Dsp1/2 | Thermo Fisher Scientific | MA1-83118 | 1:1000 |
| *AlexaFluor 594-conjugated chicken anti-rabbit IgG | Thermo Fisher Scientific | A21442 | 1:500 |
| *AlexaFluor 594-conjugated donkey anti-mouse IgG | Thermo Fisher Scientific | A21203 | 1:500 |
| *AlexaFluor 488-conjugated donkey anti-mouse IgG | Thermo Fisher Scientific | A21202 | 1:500 |
| *AlexaFluor 488-conjugated chicken anti-goat IgG | Thermo Fisher Scientific | A21467 | 1:500 |
| Hoechst33342 | Thermo Fisher Scientific | H-21492 | 1:2000 |

| Antibody used in Western blot **(*: secondary antibody)** | **Supplier** | **Product No** | **Dilution** |
|---|---|---|---|
| Mouse anti-Dsp1/2 | Thermo Fisher Scientific | MA1-83118 | 1:1000 or 1:50 |
| Mouse anti-Dsg1 | Santa Cruz Biotechnology | sc-137164 | 1:5000 |
| Rabbit anti-Dsg2 | Proteintech | 21880-1-AP | 1:1000 |
| Rabbit anti-Dsc3 | Abcam | ab126768 | 1:15000 |
| Rabbit anti-phospho-Akt (S473) | Cell Signaling Technology | 9271S | 1:500 |
| Rabbit anti-total-Akt | Cell Signaling Technology | 9272S | 1:1000 |
| Rabbit anti-Rpl7 | Bethyl Laboratories | A300-741A | 1:20000 |
| Mouse anti-β-actin | Sigma-Aldrich | A5441 | 1:20000 |
| Mouse anti-Actin | DSHB | JLA20 | 1:3000 |
| Rabbit anti-p-Histone H3 | Santa Cruz Biotechnology | SC-8656 | 1:200 |
| Rabbit anti-Active Caspase-3 | BD Pharmingen | 559565 | 1:200 |
| AlexaFluor 647-donkey anti-rabbit IgG (H+L) | Thermo Fisher Scientific | A31573 | 1:500 |
| *HRP-linked anti-mouse IgG | Cell Signaling Technology | 7076S | 1:5000 |
| *HRP-linked anti-rabbit IgG | Cell Signaling Technology | 70745 | 1:5000 |

### Example 1. Cell Reprogramming

### Example 1-1. Reprogramming of Fibroblasts

Mouse embryonic fibroblasts (hereinafter, 2° MEF) expressing Oct4, Sox2, Klf4 and c-Myc genes (hereinafter, OSKM) were prepared in the presence of doxycycline (Sigma-Aldrich, St. Louis, MO, USA).

Specifically, induced pluripotent stem cells (NG1-iPSCs) including Nanog-GFP reporter and expressing OSKM upon the administration of doxycycline were purchased from Stemgent (Beltsville, MD, USA). Then, 2°MEF was obtained from E13.5 mouse embryos derived from NG1-iPSCs through blastocyst injection. 2°MEF was maintained in MEF medium (Dulbecco's modified Eagle's medium (DMEM) supplemented with 10% FBS, 1xGlutamax, 1X MEM-non-essential amino acids (MEM-NEAA), and 1% penicillin/streptomycin). The DMEM supplemented with FBS, Glutamax, MEM-NEAA and 1% penicillin/streptomycin were all purchased from Thermo Fisher Scientific (Waltham, MA, USA). With respect to the 2°MEF, the manner described in J. Kim et al., Direct reprogramming of mouse fibroblasts to neural progenitors, Proc Natl Acad Sci USA 108, 7838-7843 (2011) was followed.

Other types of cells (4F2A MEF) expressing OSKM upon the administration of doxycycline was used by harvesting MEF from strain #011004 mouse purchased from Jacson lab. With respect to the method, B. W. Carey, et al., Single-gene transgenic mouse strains for reprogramming adult somatic cells. Nat Methods 7, 56-59 (2010) was followed.

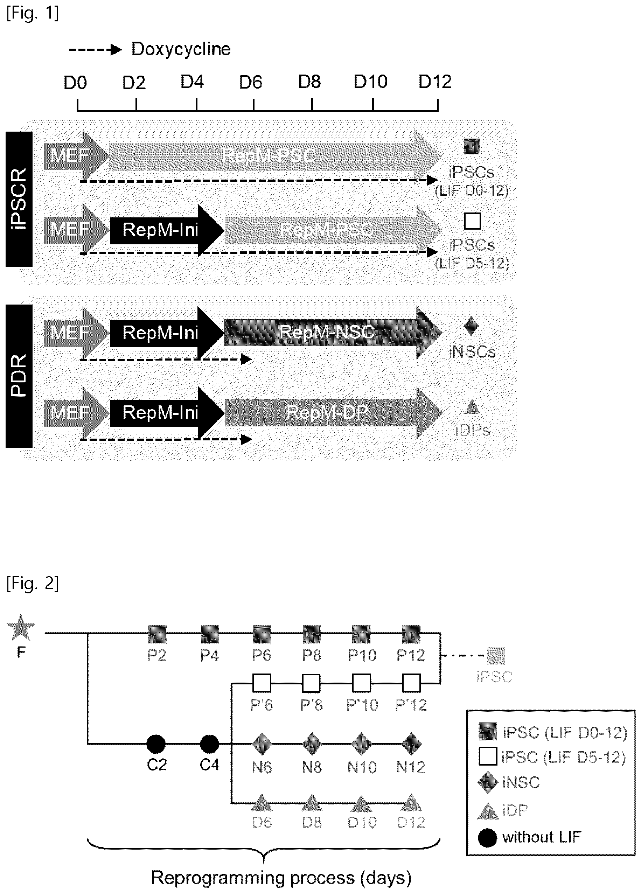

Then, in order to reprogram 2°MEF or 4F2A MEF into iPSCs, the MEF was allowed to thaw and seeded onto a plate coated with Geltrex (Thermo Fisher Scientific) at 2×10⁴ cells/cm² in the MEF medium. In this case, the induction of doxycycline began the next day. Meanwhile, two methods were used as a method (i.e., iPSCR) of reprogramming 2°MEF into iPSCs (iPSCR in FIG. 1). Specifically, the 2°MEF was further cultured in an MEF medium for one day, the culture was performed using a reprogramming initiation medium (RepM-Ini: KnockOut DMEM (Thermo Fisher Scientific), 10% KnockOut Serum Replacement (Thermo Fisher Scientific), 5% FBS, 1X Glutamax, 1X MEM-NEAA, 1% 1X Penicillin/Streptomycin and 0.055 mM β-mercaptoethanol (Thermo Fisher Scientific)) as the medium until day 4 of culture, and then the medium was replaced with an iPSC reprogramming medium for pluripotent stem cells (RepM-PSC: LIF (Millipore, Billerica, MA, USA) 1,000 U/mL) and the culture was performed until the end of culture, or the medium was replaced with RepM-PSC and then the culture was performed until the end of culture.

Meanwhile, cells having differentiation ability were prepared using a method for reprogramming 2°MEF or 4F2AMEF into neural stem cells (iNSC) or dopaminergic neural progenitor cells (iDP) using a method for inducing doxycycline only during a partial period of the culture period (PDR in FIG. 1).

RepM-Ini and an iNSC reprogramming medium for neural stem cells (RepM-NSC) were used for the iNSC reprogramming, and RepM-Ini and an iDP reprogramming medium for dopaminergic neuronal progenitor (RepM-DP) were used for the iDP reprogramming. The RepM-Ini medium is the same as described above. The RepM-NSC medium was prepared by supplementing a solution containing 0.05% BSA, 1X N2 (Thermo Fisher Scientific), 1X B27 (Thermo Fisher Scientific), 1X Glutamax, 0.11 mM β-mercaptoethanol, 20 ng/mL FGF2 (Peprotech, Rocky Hill, NJ, USA), 2 ng/mL FGF4 (Peprotech), and 20 ng/mL EGF (Peprotech) to a solution obtained by mixing advanced DMEM/F12 (Thermo Fisher Scientific) and neurobasal media (Thermo Fisher Scientific) in a ratio of 1:1. The RepM-DP medium was prepared by supplementing a solution containing 0.05% BSA, 1X N2, 1X B27, 1X Glutamax, 100 ng/mL FGF8b (Peprotection), 200 ng/mL SHH (Peprotection), and 0.11 mM β-mercaptoethanol to a solution obtained by mixing advanced DMEM/F12 (Thermo Fisher Scientific) and neurobasal media (Thermo Fisher Scientific) in a ratio of 1:1.

In order to reprogram MEF into NSCs, the MEF was allowed to thaw and seeded onto a plate coated with Geltrex (Thermo Fisher Scientific) at 2×10⁴ cells/cm² in the MEF medium. In this case, the induction of doxycycline began the next day. The MEF was further cultured in the MEF medium for one day, the culture was performed in the RepM-Ini medium until day 4 of reprogramming, and then the medium was replaced with RepM-NSC and the culture was performed in this medium until the end of reprogramming.

In order to reprogram MEF into iDPs, the MEF was allowed to thaw and seeded onto a plate coated with Geltrex (Thermo Fisher Scientific) at 2×10⁴ cells/cm² in the MEF medium. In this case, the induction of doxycycline began the next day. The 2°MEF was further cultured in the MEF medium for one day, the culture was performed in the RepM-Ini medium until day 4 of culture, and then the medium was replaced with RepM-DP and the culture was performed in this medium until the end of culture.

### Example 1-2. Cell Analysis Method in Reprogramming Process

### Microarray Analysis and Quantitative PCR

For each reprogramming process shown in FIG. 1, total RNA was harvested from each cell every other day using the RNeasy Plus mini kit and QIAshredder (Qiagen, Hilden, Germany) according to the manufacturer's manual.

In addition, the cells according to the period in which the total RNA was harvested were classified as shown in FIG. 2. The 2° MEF itself was named F, and cells under the LIF-free condition were named C, cells in the presence of LIF during the whole period for iPSC reprogramming (iPSCR) were named P, cells under the condition in which LIF was present only from day 5 of iPSCR to day 12 were named P', cells under the iNSC reprogramming condition were named N, and cells under the iDP reprogramming condition were named D, and culture day was expressed followed by P, P', N, and D.

For microarray analysis, the RNA quality of all samples was identified with the Agilent 2100 Bioanalyzer System, and amplification, labeling and hybridization steps were sequentially performed. The global gene expression profile was analyzed by the Agilent Mouse Whole Genome 4× 44K arrays (V2) chip (one-color platform; Agilent Technologies, Santa Clara, CA, USA) and all analyses were carried out in compliance with the manufacturer's protocol. Microarray data was processed using the GeneSpring software (Agilent Technologies) and normalized through global scale normalization. Functionally grouped GO terms were analyzed using the Cytoscape software platform (v3.5.1; http://www.cytoscape.org/what_is_ cytoscape.tml). Functionally grouped GO term networks were visualized using the ClueGO plug-in (v2.3.4; http://apps.cytoscape.org/apps/cluego) and CluePedia (v1.3). The ClueGO analysis of the top 24 genes was performed with 5 annotation groups (group p-value < 0.002). Biological process, cellular component, and molecular function gene enrichment were analyzed using the GSEA software (v 3.0). The GSEA variable was set to 1,000 permutations, classic enrichment statistic, and signal-to noise separation metric for each gene set. In addition, when the expression levels of the genes expressed on day 0 of reprogramming and day 6 of reprogramming were compared, the gene groups (DEG) expressed more than three times each other were analyzed.

cDNA was synthesized from 1 µg of total RNA using the iScript cDNA synthesis kit (Bio-Rad, Hercules, CA, USA). Then, the cDNA was diluted to a concentration of 1/50 and mixed with the iQ SYBR Green Supermix (Bio-Rad), and then Real-time quantitative PCR was performed using the Applied Biosystems 7500 Fast Real-Time PCR instrument system (Thermo Fisher Scientific). In this case, the cycle threshold (Ct) for each target gene was determined using software provided by the manufacturer, and the expression data was normalized with the Ct value of Rpl7 used as an internal control. Then, in order to compare fold changes between cell samples, gene expression values in each sample were compared and shown based on the MEF.

### Single Cell RNA Sequencing (scRNA-seq) Method

Single-cell RNA sequencing (scRNA-seq) was performed on a total of 33,966 cells including initiation fibroblasts obtained in the processes of iPSCR and iNSCR, iPSCs, iNSCs, and cells obtained by each culture date.

The scRNA-seq library was generated according to the manufacturer's manual using the Chromium Single Cell 3' Reagent Kit v2 (PN-120267, 10X Genomics), Chromium Single Cell A Chip Kit (PN-120236, 10X Genomics), and Chromium Single Cell i7 Multiplex Kit (PN-120262, 10X Genomics). Cell viability was estimated to be over 90% when confirmed by staining with trypan blue under a microscope. The cells were diluted to 2×10⁵ to 2×10⁶ cells/mL with DPBS containing 0.04% BSA and loaded on the Chromium microfluidic platform to capture 3,000 cells/channel. The libraries were sequenced on the Illumina HiSeq 4000 platform (2×100bp).

Raw FASTQ files were processed using default arguments and the Cell Ranger software (v2.1.0). The read was aligned with the mouse reference genome (GRCm38) at Ensembl GRCm38.92 annotation. Agene-by-cell unique molecular identifier (UMI) count matrix for each condition was generated with "expect-cells = 3,000," and aggregated into a single count matrix. Empty droplets were filtered out using the emptyDrops function of the DropletUtils (v0.99) R package under the condition of FDR ≤ 0.05. Outliers were visually inspected in the principal component analysis (PCA) plot on a quality control matrix using the scater (v1.7.18) R package, and low-quality cells with less than 1,000 UMI, less than 102.5 detected genes (103 detected genes for iNSCs), and greater than 10% of UMIs mapped to mitochondrial genes were removed. The raw count matrix was normalized by the cell-specific size factors estimated by the scran (v1.14.6) R package, and then log2-transformed with a pseudo-count of 1. Highly variable genes (HVGs) across all cells were identified using the R package. All cells were visualized in the uniform manifold approximation and projection plot (UMAP plot) using the RunUMAP function of the Seurat (v3.2.2) R package with the first 50 PCs and n.neighbors = 10. The cells between day 5 and day 7 of reprogramming were visualized in the UMAP plot from the first 25 PCs. The cells were clustered using the FindClusters function of the R package with the first 30 PCs and resolution = 0.6. Gene set signature scores were calculated using the AddModuleScore function of the Seurat and conventionally known marker genes. The blastema-specific markers described in W. Wang et al., Changes in regeneration-responsive enhancers shape regenerative capacities in vertebrates. Science 369, (2020) were used to calculate the blastema signature scores. The desmosome (GO:CC) and epithelial cell differentiation (EMT, Hallmark) gene list was obtained from MSigDB.

Pathways in the UMAP space were analyzed using the slingshot function of the slingshot (v1.4.0) R package with a predefined initiation cluster (Day 5-7: 1, Day 2-8: 3, zebrafish: 6, axolotl: 7). The signature score along the pathways was softened by lowering the regression, and in the WOT analysis, the transport map was performed with optimal_transport command-line interface and default argument on HVG-expression data using the wot (v1.0.8) python package.

### Methods of Alkaline Phosphatase (AP) Staining and Immunofluorescence Staining

For AP staining, each cell was washed once with Dulbecco's phosphate-buffered saline (DPBS, WELGENE, Daegu, Korea) and fixed with a 10% formalin solution (Sigma-Aldrich) for 30 seconds. The AP staining was performed using a leukocyte AP kit (Sigma-Aldrich) according to the manual of Sigma-Aldrich. The fixed sample was washed once and allowed to stand with the AP substrate solution for 20 minutes in the dark.

For immunofluorescence staining, each cell was washed with DPBS, fixed and blocked with DPBS containing 4% paraformaldehyde (Electron Microscopy Sciences, Hatfield, PA, USA) and 0.15% picric acid (Sigma-Aldrich) for 15 minutes, and permeabilized with DPBS containing 3% BSA (Thermo Fisher Scientific) and 0.3% Triton X-100 (Sigma-Aldrich) for 1 hour at room temperature. The primary antibodies were diluted in 1% BSA and allowed to stand overnight at 4 °C. After repeated washing with DPBS containing 1% BSA, all samples were allowed to stand at room temperature for 1 hour with Alexa-594- or Alexa-488-conjugated secondary antibodies (Thermo Fisher Scientific). All fluorescence images were obtained with the Axio Vert. A1 microscope (Carl Zeiss, Oberkochen, Germany) and the Evos FL auto 2 imaging system (Thermo Fisher Scientific).

### Transmission Electron Microscopy Imaging

To analyze desmosome formation, reprogramming was performed on geltrex coated coverslips (Electron Microscopy Sciences, Hatfield, PA, USA). Cells were fixed for 1 hour in a 0.1 M cacodylate solution (pH 7.0) containing 2.5% glutaraldehyde. Thereafter, the cells were treated with 2% osmium tetroxide at 4 °C for 2 hours. The cells were dehydrated with graded acetone and then embedded in Spurr Media (Electron Microscopy Services). Each sample was sectioned by 60 nm with ultra-microtome (RMC MTXL; Boeckeler Instruments, Tuscon, AZ, USA), stained with 2% uranyl acetate for 20 minutes, and stained with lead citrate for 10 minutes. Each section was observed with a H-7600 (HITACHI, Tokyo, Japan) transmission electron microscope at 80 kV.

### Example 1-3. Changes in Cells for Each Reprogramming Method and Each Reprogramming Period

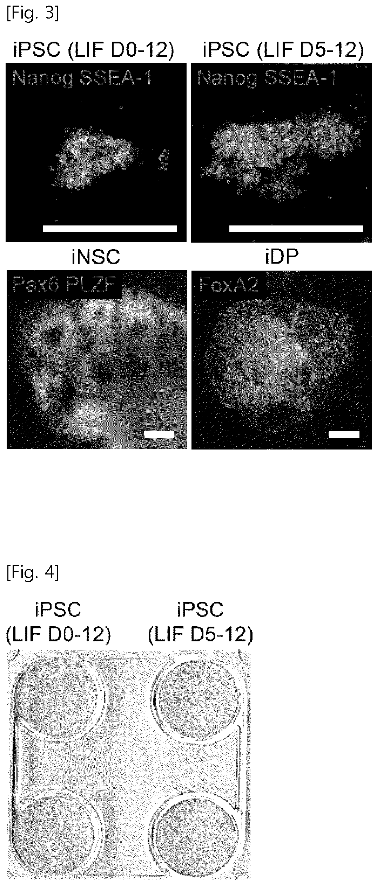

According to immunostaining image analysis of iPSC-specific markers Nanog and SSEA-1, iNSC-specific markers Pax6 and PLZF, and iDP-specific marker FoxA2 for cells on day 12 of reprogramming, cells on day 12 of reprogramming showed proper conversion of the fate of the cells to iPSCs, iNSCs, and iDPs (FIG. 3).

In addition, as a result of AP staining for iPSCs on day 12 of reprogramming, it was confirmed that the presence or absence of LIF did not have any effect on the process of reprogramming 2° MEF into iPSCs (FIG. 4).

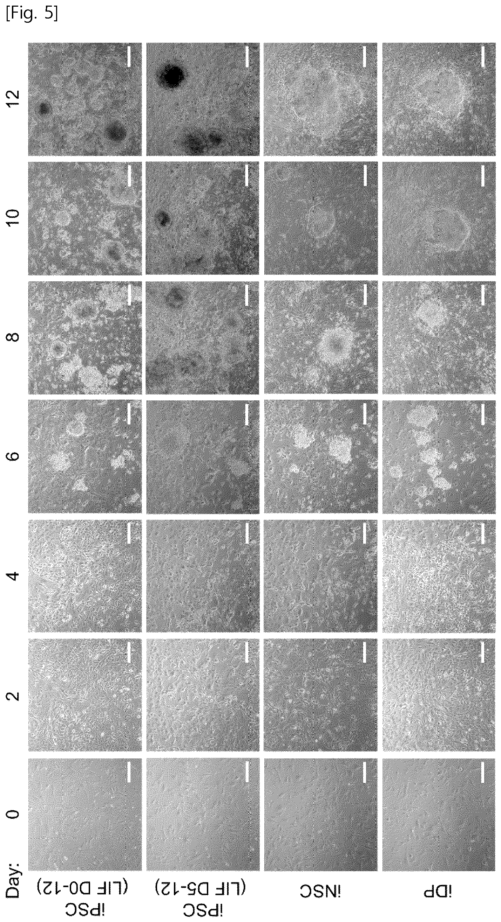

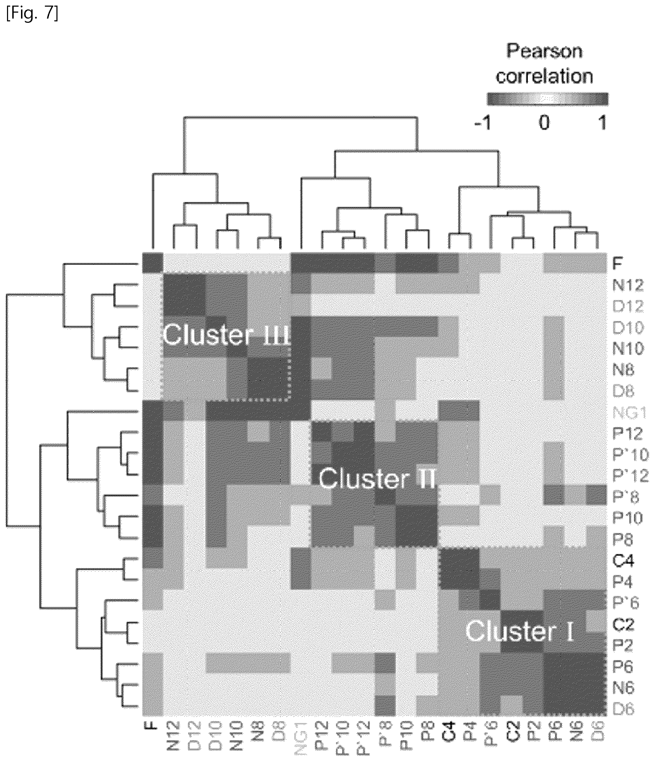

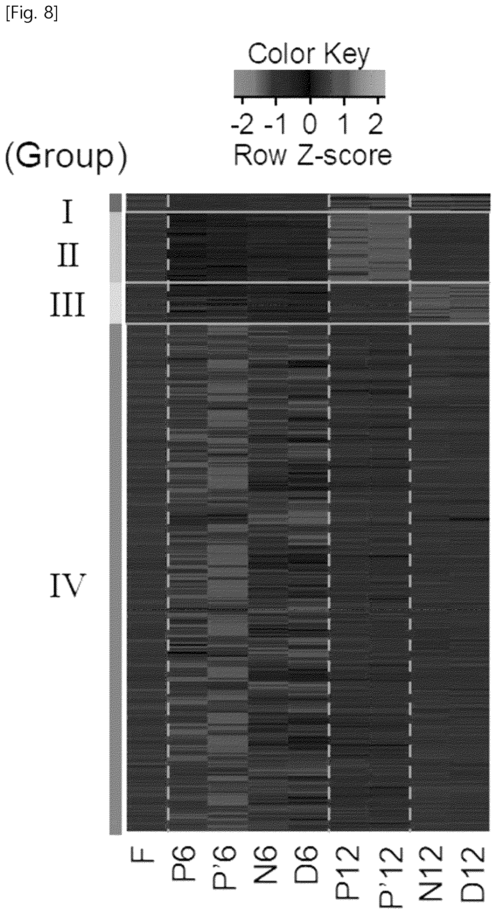

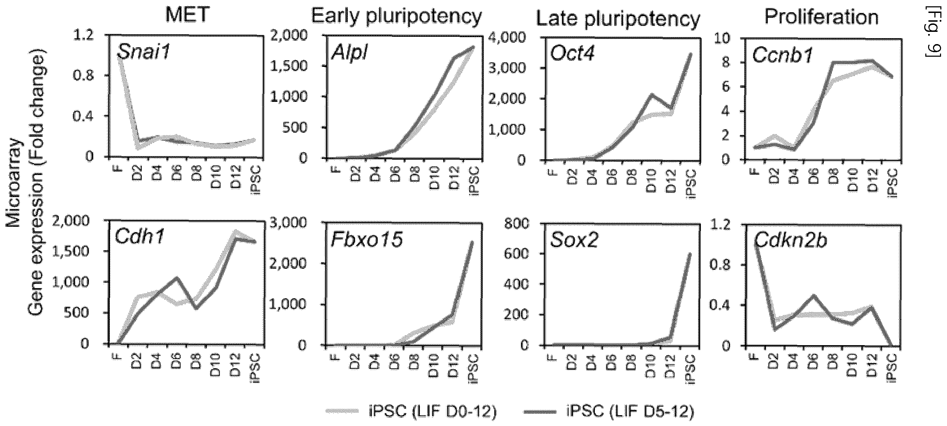

Furthermore, as a result of analyzing the phase-contrast images and the immunofluorescence staining images from day 0 to day 12 of reprogramming, it was found that there was little difference in cells according to the reprogramming method up to day 6 of reprogramming (FIG. 5). In addition, transcriptomic similarity analyzed by principal component analysis (PCA) showed that all cells were divided into the same group until day 6 of reprogramming (cluster I in FIG. 6). As a result of analyzing microarray data for each cell shown in FIG. 2 with hierarchical clustering, it was confirmed that iPSCR and PDR were reprogramming methods distinguished from each other, but reprogram cells through a common biological pathway until day 6 of reprogramming (FIGS. 7 and 8). Furthermore, since all of the cells on day 6 of reprogramming lost their characteristics as somatic cells and the pluripotency-related genes were not yet activated, day 6 of reprogramming was classified as a kind of "intermediate-phase" (FIG. 9).

Genes that contribute to the intermediate stage on day 6 of reprogramming were analyzed through the DEG analysis of Examples 1-2. DEGs were classified into four groups through k-means clustering, and classification criteria are shown in FIG. 10 (bottom of FIG. 10). The gene corresponding to Group IV among these showed intermediate stagespecific expression of more than 70% of the analyzed gene (FIG. 8 and top of FIG. 10).

In addition, network-based Gene Ontology analysis was performed using ClueGO for 244 genes common in Group IV of iPSCR and Group IV of PDR. These 244 genes were rarely expressed in fibroblasts, iPSCs, iNSCs, and iDPs, but the expression level was found high on day 6 of reprogramming (FIGS. 11 and 12). The GO term located at the top in the GO analysis for these genes is shown in FIG. 13.

Meanwhile, when the cells on day 6 of culture, the cells in which the reprogramming began, and the final cells in which the reprogramming was completed were analyzed with the Gene Set Enrichment Analysis, gene set that had the best ranking was found to be related to desmosome, and in addition, it was found to be related to epithelial cell differentiation (FIG. 14). Thus, it was confirmed that the epidermis-related genes have the characteristics related to desmosome and epithelial cell differentiation which are "intermediate-stage" specific in both iPSCR and PDR.

When the presence of desmosome formation was observed with a transmission electron microscope, mature desmosome was formed only in cells on day 6 of reprogramming (FIGS. 15 and 16).

### Example 1-4. Correlation between Desmosome and Reprogramming Methods

Mesenchymal-to-epithelial transition (MET) is an essential process in the early stage of reprogramming. Accordingly, it was confirmed whether mesenchymal-related genes and epithelial-related genes were expressed (FIG. 17). Increased expression of genes unrelated to desmosome (Cdh1, Ep-cam, and Cldn6) among epithelial genes was observed in cells on day 6 of reprogramming and iPSCs. On the other hand, decreased expression of mesenchymal genes (Snail, Slug, Zeb1, Twist1, Twist2, Cdh2, and Fn) was shown in all cells. However, it was found that the expression of desmosomal component genes (*Dsp* and *Pkp1*) and cytokeratins (*Krt8* and *Krt19*) were increased in cells on day 6 of culture, while decreased in the iPSCs. This suggests that the desmosome-related epithelial gene is regulated differently from the epithelial gene involved in MET (*e.g.*, Cdh1).

In addition, except for desmoglein2 (Dsg2), which is mainly expressed in the iPSCs, it was found that some desmosome proteins were specifically expressed in cells on day 6 of reprogramming (FIGS. 18A to 18C). Most desmosome proteins were increased temporarily during reprogramming, and decreased in the iPSCs.

### Example 1-5. Correlation between Cells to Be Reprogrammed and Reprogramming Method and Desmosome

It was found that the reprogramming method and the cell type to be reprogrammed did not affect the expression pattern of desmosome proteins on day 6 of reprogramming.

In order to confirm whether the expression pattern of desmosome proteins changes in the process of converting human bronchial epithelial cells (NHBEs) and prostate epithelial cells (PrECs) into iPSCs, a normalized microarray matrix of accession number GSE50206 was obtained from NCBI Gene Expression Omnibus (GEO) and analyzed using the GEO function of the GEOquery (v.2.58.0) R package.

In addition, in order to confirm whether the expression pattern of desmosome proteins changes in the process of converting 2°B cells into iPSCs by treating the 2°B cells with OSKM, the microarray data provided by B. Di Stefano et al., C/EBPalpha poises B cells for rapid reprogramming into induced pluripotent stem cells, Nature 506, 235-239 (2014) was analyzed.

As a result of analyzing data related to 2° B cell reprogramming, temporary desmosomal component activation was found in the process of converting the 2° B cells, which are non-pluripotent cells, into iPSCs (FIG. 19A). In addition, similar results were observed in the processes of NHBE and PrEC reprogramming (FIG. 19B). This shows that desmosome formation is a major event in the "intermediate stage" of OSKM-related reprogramming and occurs regardless of the types of cells to be reprogrammed and reprogramming method.

### Example 1-6. Dsp Role in Reprogramming (I)

A DSP protein is an essential factor in the formation of mature desmosome, linking intermediate filament to desmosomal plaque. In order to determine whether mature desmosome formation is essential for reprogramming by OSKM, β-D-1-thiogalactopyranoside (IPTG)-inducible short hairpin RNA (sh*Dsp*) for the dsp was used in the processes of iPSCR and iNSCR to inhibit Dsp expression (FIG. 20A).

ApLKO-GFP-IPTG-3xLacO lentiviral vector containing shRNA (SEQ ID NO: 85: GCCTACAAGAAAGGTCTCATT) for the mouse *Dsp* gene was prepared by requesting Sigma-Aldrich. In this case, shCTRL (sigma Aldrich, SCH332), which is a plasmid vector containing the off-target shRNA, was used as a control group. Lentiviral vectors were produced using HEK 293T cells, and the cells were transfected with the prepared lentiviral vectors together with vesicular stomatitis virus coat protein plasmid (pMD2.G; Addgene, Watertown, MA, USA) and the packaging plasmid (psPAX2; Addgene) by using TransIT-2020 transfection reagents (Mirus Bio, Madison, WI, USA). After 8 hours to 16 hours, the transfected HEK 293T cells were washed with DPBS and then further cultured in the MEF medium for another 48 hours. After 48 hours, the supernatant of the medium was collected, and the supernatant of the virus, from which HEK293T cells were removed through centrifugation and filtration, was collected. Then, the collected viral supernatants were used to transfect the 2° MEF with 6 µg/mL of polybrene (Sigma-Aldrich).

The transfected 2° MEF was dissociated at 0.05% trypsin-EDTA for 5 minutes, washed with DPBS, then resuspended in FACS buffer (DPBS containing 0.5% BSA), and isolated using the FACSaria cell sorter (BD Biosciences). The selected transfected 2°MEF was seeded onto a geltrex coated plate at 2×10⁴ cells/cm². For knockdown of the *Dsp* gene, the cells were treated with 1 mM of IPTG (Sigma-Aldrich) for 6 days from the day following transfection.

The *Dsp* knockdown (*Dsp* KD) reduced the efficiency of iPSC production and showed a significant decrease in the number of colonies expressing the pluripotent biomarkers (alkaline phosphatase and Nanog) in cells (FIGS. 20B and 20C). The *Dsp* KD also significantly reduced the resulting iNSCs (FIG. 20D).

As a result, as shown in FIG. 20, the *Dsp* knockdown reduced the formation of gIC (FIGS. 20E and 20F). Accordingly, it was confirmed that the expression of the *Dsp* gene was an important factor involved in the formation of gIC. In addition, it was found that the *Dsp* knockdown interferes with the Akt signaling (FIG. 21).

### Example 1-7. Dsp Role in Reprogramming (II)

In the process of reprogramming human fibroblasts by means of the iPSCR or iNSCR method used in Example 1-1, DSP was overexpressed.

CRL-2097 (American Type Culture Collection, Manassas, VA, USA), a human fibroblast, was plated in a 24-well plate at 30,000 cells/cm² and transformed using a Sendai virus (SeV) mixture (CytoTune^{™}-iPS 2.0 Sendai reprogramming kit; Thermo Fisher Scientific) according to the manufacturer's manual. The next day, the SeV mixture was washed with DPBS.

For human iPSC reprogramming, the cells were cultured for another two days in a fibroblast medium (10% FBS, 1X Sodium pyruvate (Thermo Fisher Scientific), and MEM medium (Thermo Fisher Scientific) to which 1X MEM-NEAAwas added). On day 3 after the transformation, the fibroblast medium was replaced with an iPSC reprogramming medium (3.0 µM CHIR99021 (Tocris Bioscience, Bristol, UK), 0.5 µM A83-01 (Tokris), and mTeSR-1 (STEMCELL Technologies, Vancouver, Canada) medium to which 0.2 mM NaB (Sigma-Aldrich) was added).

For human iNSC reprogramming, the SeV mixture was washed with DPBS, the medium was replaced with a human neurological reprogramming medium to which 3.0 µM CHIR99021, 0.5 µM A83-01, and 10 ng/mL hLIF (Peprotection) were added, and the human neurological reprogramming medium was replaced every other day.

On day 4 of the transformation, cells were transfected with 1 µg of DSP plasmid (Addgene, Cat. 32227) and an empty plasmid (pEGFP-N1; Takara Bio, Shiga, Japan).

The overexpression of DSP significantly increased the efficiency of iPSC and iNSC production (FIGS. 22A to 22E). This shows that the Dsp is a major factor having an effect on the progress and intermediate phase of reprogramming by OSKM.

### Example 2. Dsp Gene Expression Inhibition Experiment (in vivo)

Reptiles and fish can regenerate damaged sites by dedifferentiation of cells in the body's damaged sites to form a blastema. The blastema is a dedifferentiated multipotent cell population, and is redifferentiated into lineage-specific cells that constitute damaged tissues or organs while maintaining the memory of the derived tissue. Meanwhile, since a temporary increase in Oct4, Sox2, Klf4, and c-Myc was observed at the time of development of blastemal cells, reprogramming of Example 1 and tissue regeneration in reptiles and fish were expected to have a common mechanism.

Accordingly, an experiment was conducted on zebrafish to confirm the role of the *Dsp* gene. The zebrafish (Danio rerio, AB wild type) were acclimated at 28.5 °C under standard conditions. The zebrafish were fed on dry food and brine shrimp every day and raised in a schedule for 14 hours in a bright place and 10 hours in a dark place. The rearing and animal care of the zebrafish was carried out in accordance with guidelines from the KRIBB and approved by the KRIBB-IACUC (Approval No.: KRIBB-AEC-17073, KRIBB-AEC-20056).

### Example 2-1. Confirmation of Reduction in Blastemal Cells due to Inhibition of Dsp Gene Expression

The embryos of the wild-type zebrafish were maintained in E3 egg water (5 mM NaCl, 0.17 mM KCl, 0.33 mM CaCl₂, 0.33 mM MgSO₄) at 28.5 ° C in a petri dish. 1 nL of a 0.4 mM control morpholino solution (ctrl MO) or a 0.4 mM mixed morpholino solution of *dspa* and *dspb* (dsp mix MO) was injected into one-cell eggs. The zebrafish larvae at 2 day postfertilization (dpf) were anesthetized with tricaines in egg water and then the tail fins were amputated using a surgical razor blade. As the fin amputated sites, the same sites just posterior to notochords was amputated. After 21 hours, the fin-amputated sites were fixed, and the *junbb* gene, which is a blastemal cell marker gene, was confirmed by whole mounting in situ hybridization (WISH).

Specifically, first, to make an *in situ* probe, PCR was used to amplify the DNA template for the *junbb* gene, a blastemal cell marker gene, in cDNA of the wild-type zebrafish. The probe used in this case is shown in Table 4 below.

**[Table 4]**

| **Gene** | **Forward probe** | **SEQ ID NO** | **Reverse probe** | **SEQ ID NO** |
|---|---|---|---|---|
| *junbb* | TGGGTTACGGTCACAACGAC | 86 | CAGTGTCCGTTCTCTTCCGT | 87 |

Then, the PCR product was separated and purified on agarose gel, and cloned into the pCR^{™} Blunt II-TOPO^{®} vector. Antisense probes labeled with Dig were transcribed *in vitro* by the SP6 or T7 RNA polymerase kit (Roche), and purified with the NucAway spin column (Thermo Fisher Scientific).

The fins of adult and larva zebrafish for WISH were fixed in 1X PBS solution with 4% paraformaldehyde and dehydrated using methanol. Then, it was stored at -20 °C for 30 minutes and prepared by continuously rehydrating with a 1X PBST solution. The rehydrated embryos were treated with protease K at 1X PBS and then fixed with 4% paraformaldehyde. The antisense probes were hybridized with fixed embryos at each developmental stage in a hybridization solution (adjusted to pH 6.0 using 5 mg/mL Torula yeast RNA type VI, 50 µg/mL heparin, 50% formamide, 5X SSC, 0.1% Tween-20, 1M citric acid) overnight at 70 °C. The probes were continuously washed using 2X SSCT-F (2X SSCT, 50% formamide, 0.1% Tween20), 2X SSCT (2X SSCT, 0.1% Tween20), 0.2X SSCT (0.2X SSCT, 0.1% Tween20) at 70 °C, and 1X PBST at room temperature.

Thereafter, non-specific binding was blocked with a blocking solution (5% horse serum, 1X PBST) at room temperature, and alkaline phosphatase-binding anti-digoxigenin antibodies (Roche) were added thereto to react overnight at 4 °C. An NBT/BCIP solution (Roche) was used as an alkaline phosphatase (AP) substrate in order to detect the expression signal of the transcriptome. Expression patterns of transcriptomes were observed using the Olympus SZX16 microscope and imaged with the TUCSEN Dhyana 400DC49. Quantification of WISH was performed according to the AP activity of the fin fold. For gene expression calculation, data was pixelated using the Photoshop (Adobe Systems Inc., San Jose, CA, USA) and the number of AP positive (blue) pixels was calculated using the Image J software. In this case, Hpf stands for "hours post-fertilization," and Hpa stands for "hours post-amputation."

As a result, when ctrl MO was injected, it was confirmed that the *junbb* gene was marking the blastemal cells by *forming junbb*(+) cells at the amputation site. On the other hand, when dsp mix MO was injected, it was confirmed that the formation of *junbb*(+) cells was reduced, and thus it was confirmed that the formation of blastemal cells was inhibited due to the inhibition of the *Dsp* gene expression (FIG. 23B).

### Example 2-2. Confirmation of Decrease in Expression of Blastemal Cell Marker Gene due to Inhibition of Dsp Gene Expression

The embryos of the wild-type zebrafish were maintained in E3 egg water (5 mM NaCl, 0.17 mM KCl, 0.33 mM CaCl₂, 0.33 mM MgSO₄) at 28.5 ° C in a petri dish. 1 nL of a 0.4 mM control morpholino solution (ctrl MO) or a 0.4 mM mixed morpholino solution of *dspa* and *dspb* (dsp mix MO) was injected into single-cell eggs. The zebrafish larvae at 2 dpf were anesthetized with tricaines in egg water and then the tail fins were amputated using a surgical razor blade. As the fin amputated sites, the same sites just posterior to notochords was amputated.

The zebrafish embryos at each stage were obtained using the TRI reagent (Thermo Fisher Scientific), total RNA was then extracted with the Direct-zol RNA miniprep kit (Zymo Research, Irvine, CA, USA), and cDNA was synthesized using the SuperScript III First-Strand Synthesis System (Thermo Fisher Scientific). The synthesized cDNA was amplified through semi-quantitative PCR using the primers shown in Table 2 above.

As a result, it was confirmed that the *msx1b, fgf20a, her6,* and *hspd1* genes were expressed in the wild-type zebrafish, whereas the expression of these genes was significantly reduced in the zebrafish in which the *dsp* gene was knocked down (FIG. 24). Accordingly, it was confirmed that the expression of the blastemal cell marker genes, which are important for zebrafish's fin regeneration, decreases when the *dsp* gene was knocked down.

### Example 2-3. Confirmation of Decrease in Fin Regeneration Ability due to Decrease in Blastemal Cell Formation

In Example 2-1, it was confirmed that the blastemal cells were reduced due to the inhibition of the *dsp* gene expression. The blastemal cells are important cells involved in the cell regeneration. In order to confirm whether the ability to regenerate the fin is reduced due to the decrease in the formation of the blastemal cells, the regeneration ability of the fin was measured after inhibiting the expression of the *dsp* gene. The tail fin regeneration experiment was performed on adult zebrafish aged 6 to 9 months. The adult zebrafish was acclimated at 33 °C before the experiment. The zebrafish were anesthetized with tricaine before amputating the tail fins, and the tail fins were amputated at seven bony segments distal to the fin girdle. After tail amputation, the zebrafish were returned to the 33 °C tank. To knock down the *Dsp* gene, morpholino oligonucleotides (Gene Tools Inc., Philomath, OR, USA, hereinafter, morpholino) in Table 5 below were used.

**[Table 5]**

| Morpholino | Sequence information | SEQ ID NO |
|---|---|---|
| *pou5f3* MO | 5'-CGCTCTCTCCGTCATCTTTCCGCTA | 88 |
| *dspa* MO | 5'-AAACTAAAACCGAGGCTGACCTTCT | 89 |
| *dspb* MO | 5'-CTGACTGTGTTTCAGACTGACCTGT | 90 |

Each morpholino contains a 3-fluorescein tag and is resuspended in water. The morpholinos of *dspa* and *dspb* were mixed in a ratio of 1:1 to knock down both desmoplakin genes simultaneously. At 48 hours post-amputation, 1 mM morpholinos were injected into the regeneration tissue on the dorsal side of each zebrafish tail fin using the PV380 pneumatic picopump (World Precision Instruments, Sarasota, FL, USA). Each morpholino injection was targeted to each bone regeneration tissue, and approximately 70 nL of a morpholino solution was injected.

Immediately after injection of the morpholino solution, electroporation was performed on both the dorsal and ventral (to control non-specific electroporation effects) sides of the fin using NEPA21 Electroporator (Nepa Gene Co., Ltd., Chiba, Japan). In this case, the electroporation parameter used three consecutive 50-msec pulses at 15 V with a 50-msec pause between pulses using a CUY647 15-mm-diameter platinum pate electrode (Protech International Inc., Cornelius, NC, USA). In order to increase the efficiency of electroporation, electroporation was performed twice using the same parameters. The zebrafish was then returned in the water tank at 33 °C. At 72 hours post-amputation, each fin was photographed using an Olympus SZX16 microscope equipped with a TUCSEN Dhyana 400DC digital camera.

The regeneration area of the dorsal and ventral fins was calculated using Image J software (National Institutes of Health, Bethesda, MD, USA). In this case, the percentage of regeneration was calculated by ((D3dpa - D2dpa) / (V3dpa - V2dpa)) X 100, and the statistical significance of morpholino on regeneration was analyzed using Student's t-test.

As a result, as shown in FIG. 23, it was confirmed that the fin regeneration ability of zebrafish was reduced when the *Dsp* gene was knocked down.

### Example 3. Identification of Pluripotent Cells and Characteristics thereof

### Example 3-1. Identification and Analysis of Cell Aggregates Formed in "Intermediate Phase" of Reprogramming

On day 6 of reprogramming, cell aggregates were formed (FIGS. 5 and 20), and the number of these cell aggregates decreased when Dsp expression was suppressed (FIGS. 20E and 20F).

The cells were dissociated for 1 hour using Accutase (MilliporeSigma, Billerica, MA, United States) to isolate the cells on day 6 of reprogramming from the plate. In addition, after the supernatants on day 6 of reprogramming were separated, the separated supernatants were centrifuged at 300X g for 3 minutes, and washed with DPBS (WELGENE, Daegu, Korea), and gICs were isolated.

Cell aggregates were stained with antibodies against Dsp and antibodies against Dsc3 (FIGS. 25A and 25B). As a result of the analysis, it was found that the cell aggregates formed on day 6 of culture were classified into two cell groups for Dsp and Dsc3. That is, the cell aggregates were classified into "desmosomal-component expressing intermediate cells (dICs)," which is a cell population in which both Dsp and Dsc3 are expressed, and "granular intermediate cells (gICs)," which is a cell population showing a granular morphology without expressing Dsp and Dsc3. The two types of cell populations were observed together in one cell aggregate, and gICs were not formed during *Dsp* KD, even though the gICs did not express Dsp (FIGS. 20E, 20F, 25A, and 25B). Furthermore, retrospective tracing from the established colonies by iPSCR and iNPCR supported the same hierarchical relationship between dICs and gICs (FIG. 26), and thus, it was confirmed that Dsp expression was a condition required for gIC formation.

### Example 3-2. Analysis of dICs and gICs

Since dICs and gICs were observed on day 6 of reprogramming, cells present on day 5 of reprogramming and day 7 of reprogramming were analyzed (FIGS. 27A and 28B). Similar cellular characteristics were shown in both iPSCR and iNSCR until day 6 of reprogramming, but *Shisa8,* the marker of the successful MET during iPSC reprogramming, was expressed in a subpopulation of the cell population on day 5 and day 7 of reprogramming (FIGS. 27A and 27B). In addition, the cells that appeared to be *Shisa8*+/*Dsp*+ on the UMAP were dICs, and the cells that appeared to be *Shisa8*+/*Dsp*- on the UMAP were gICs (FIGS. 27B and 27C).

In fact, in the isolated gICs, *Dsp* expression remained low, but *Shisa8* and *Cdk1* were highly expressed (FIG. 27E).

In addition, gICs was easily detached from the culture dish, and formed iPSC-like colonies when the iPSCR process was started again after reattachment (FIG. 27D). Although the gICs have the potential to become iPSCs, the gICs rarely expressed pluripotency markers (Nanog, Rex1, and Esrrb), and thus it was confirmed that the gICs are distinct cells from iPSCs (FIGS. 27E and 27C).

In addition, it was confirmed that the *Spink2* gene was not expressed in iPSCs, whereas the expression thereof was at a considerable level in the gICs (FIG. 29). Thus, it was confirmed that gICs are cells having characteristics different from iPSCs.

To investigate the lineage relationship between the dICs and gICs, Slingshot analysis (the analysis method disclosed in K. Street et al., Slingshot: cell lineage and pseudotime inference for single-cell transcriptomics. BMC Genomics 19, 477 (2018)) was performed. As a result of the analysis, it was found that the dIC populations (clusters 1, 4, and 8; *Shisa8*+/*Dsp*+) were bifurcated into two trajectories by *Shisa8* expression (FIGS. 27F and 28D). Cells along the *Shisa8*-negative path (clusters 2, 6, 7, and 11) expressed stromal signatures such as MEF identity and senescence, teaching that these cells are resistant to reprogramming (FIGS. 27C, 25E, and 28A).

The dICs along the successful reprogramming path as the *Shisa8*-positive path were converted into the gIC populations (clusters 0, 3, and 12; *Shisa8*+/*Dsp*-). Waddington optimal transport (WOT) analysis (the analysis method is disclosed in G. Schiebinger et al., Optimal-Transport Analysis of Single-Cell Gene Expression Identifies Developmental Trajectories in Reprogramming. Cell 176, 928-943 e922 (2019)) showed the same results, confirming that gICs are derived from dICs (FIGS. 28E and 28F). Thus, it was confirmed that in the Shisa8-positive successful reprogramming path, Dsp expression is required to establish the dICs, and then Dsp expression is reduced to establish the gICs.

Furthermore, it was found that when reprogramming was interrupted using MK2206, which is an inhibitor of Akt signaling, the number of cell aggregates was significantly reduced, butMK2206 did not affect the self-renewal of iPSCs and iNSCs (FIGS. 30Ato 30I). Therefore, it was confirmed that Dsp-Akt signaling is a specific and essential factor for the generation of dICs and gICs.

In addition, even when MK2206 was used, iPSCs were not established in the intermediate phase, and even when scRNA-seq data was analyzed, no cells expressing the pluripotency gene expressed by iPSCs in the intermediate phase were detected. Since MK2206 does not interfere with the self-renewal of iPSCs, it was confirmed that cell aggregates that appear transiently in the intermediate phase of the reprogramming process do not contain iPSCs.

Consequently, it was confirmed that dICs and gICs are essential cells for the progress of cellular reprogramming.

### Example 3-3. Real time PCR Analysis

gIC specific marker gene expression analysis was performed using the Real time PCR analysis used in Example 1-2, and the primers used are shown in Table 1 above.

As a result, it was confirmed that the *Shisa3, Foxo4, Ptp4a3, Blvra, Mbnl3, Mthfd2,* and *Dctpp1* genes were expressed at considerable levels in gICs, whereas the expression of these genes was very low in iPSCs (FIG. 31). Thus, it was once again confirmed that gICs are cells having different characteristics from iPSCs.

### Example 3-4. Differentiation Potential of gICs into Blastemal Cells

The gICs were seeded into ultralow attachment 96-well plates (Corning Incorporated, Kennebunk, ME, United States) and then cultured in RepM-Ini medium containing doxycycline for 24 to 48 hours. Then, the gIC aggregates were transferred to 35-mm petri dishes, and suspension-cultured in the RepM-Ini medium without doxycycline for 7 days. Thereafter, the aggregates were attached to the geltrex-coated culture plates, and then cultured for another 7 days. Immunofluorescence staining was performed by the immunofluorescence staining method described in Example 1-2 to confirm the differentiation potential of gICs into three germ layers. In this case, the primary and secondary antibodies used are shown in Table 3 above.

As a result, as shown in FIG. 32, it was confirmed that the gICs can be naturally differentiated into various cells constituting three germ layers without specific differentiation conditions.

### Example 3-5. Changes of gICs into iPSCs and iNSCs

gICs were seeded into geltrex-coated plates at 2×10⁴ cells/cm² and cultured in the RepM-Ini medium containing doxycycline for 2 days. Thereafter, the culture medium was replaced with RepM-PSC containing doxycycline or RepM-NSC without doxycycline, and then cultured for 4 days.

Then, the cultured cells were washed once with DPBS. The cells were fixed with 4% paraformaldehyde (Electron Microscopy Sciences, Hatfield, PA, USA) containing 0.15% picric acid (Sigma-Aldrich) for 15 minutes. Thereafter, washing was carried out three times with DPBS, followed by reaction in 3% BSA (Thermo Fisher Scientific) containing 0.3% Triton X-100 (Sigma-Aldrich) for 1 hour at room temperature. The cells were treated with the primary antibodies diluted with 1% BSA and reacted overnight at 4 °C. The next day, after washing three times with DPBS, the reaction was carried out at room temperature for 1 hour by treatment with Alexa-594 or Alexa-488-labeled secondary antibodies (Thermo Fisher Scientific). After washing three times with DPBS, fluorescence images were captured using the Axio Vert.A1 microscope (Carl Zeiss, Oberkochen, Germany) and Evos FL auto 2 imaging system (Thermo Fisher Scientific). In this case, the primary antibodies used are shown in Table 3 above.

As a result, as shown in FIGS. 26 and 33, it was confirmed that iPSCs and neural stem cells (iNSCs) were formed from gICs.

### Example 3-6. Experiment on Tumorigenicity of gICs (in vivo)

The rearing and animal care of mice was carried out in accordance with guidelines from the KRIBB and approved by KRIBB-IACUC (Approval No.: KRIBB-AEC-20062).

To confirm the tumorigenicity of gICs, gICs with 5×10³, 5×10⁴ or 2×10⁵ cells and iPSCs with 5×10³ or 5×10⁴ cells were injected subcutaneously into severe combined immunodeficient mice (Jackson Laboratory, total of 12 mice) in which obesity and diabetes are caused. Thereafter, the mice were monitored for 4-8 weeks. In this case, iPSCs with tumorigenicity were used as a positive control.

As a result, as shown in FIG. 34, it was confirmed with the naked eyes that tumors were formed in the case of mice administered iPSCs at week 4 after transplantation. On the other hand, in the case of mice administered gICs, it was confirmed with the naked eyes that tumors were hardly formed, except for one mouse, in the group in which the most cells were added, which were 2×10⁵ cells. In addition, as a result of measuring the degree of tumor formation of mice, in the case of mice (D, E) administered iPSCs, all tumors were formed at week 4, whereas in the case of mice (A) administered gICs with 2×10⁵ cells, only about 20% of tumors were formed, and no tumors were formed in mice (B, C) administered gICs with 5×10³ and 5×10⁴ cells. Thereafter, when the mice administered gICs were observed up to week 8, about 50% of tumors were formed in the case of mice (A) administered gICs with 2×10⁵ cells and about 20% in the case of mice (B) administered gICs with 5×10⁴ cells, and no tumors were formed in the case of mice (C) administered gICs with 5×10³ cells. Therefore, it was confirmed that gICs are pluripotent cells which have significantly lower tumorigenicity than iPSCs.

### Example 4. Tissue Regeneration Effect of gICs

By treating the skin of the mouse model, in which the wound was caused, with gICs, it was confirmed whether gICs actually showed a tissue regeneration effect.

After performing peritoneal injection of 80 µL of 3% Avatin on one healthy 30-week-old ICR mouse and anesthetizing the mouse, the hair in the rear of the spine was shaved with an electric hair clipper, two circular wounds with a radius of 5 mm were made to a certain depth up to the fascia layer, one wound was treated with nothing (control wound), and the other wound was treated with gICs (experimental group wound). After treating the surface of the wound in the experimental group with 4.5×10⁴ gICs, the cross-sectional areas of the wound on days 3, 6, 13, and 16 after causing the wound (*i.e*., 3 day-post-injury (d.p.i), 6 d.p.i, 13 d.p.i, and 16 d.p.i) were checked to determine how much the wound was cured. For each period, the ratio of the area in which the circular wound still remains to the cross-sectional area of the first circular wound (residual wound ratio) and the wound healing ratio are indicated as the residual wound ratio/wound healing ratio in Table 6 below.

**[Table 6]**

| | 3 d.p.i. | 6.d.p.i. | 13. d.p.i. | 16 d.p.i |
|---|---|---|---|---|
| Control group | 66%/34% | 48%/52% | 25%/75% | 20%/80% |
| Experimental group | 34%/66% | 27%/73% | 13%/87% | 4%/96% |

As shown in Table 6, the wound treated with gICs showed a fast wound healing effect, and a remarkably excellent effect of regenerating the skin tissue. Therefore, it was confirmed that gICs have an excellent effect of regenerating tissue.

## Claims

1. A method for converting non-pluripotent cells into pluripotent cells, the method comprising:
inducing the non-pluripotent cells to express reprogramming factors, thereby overexpressing desmosome-related genes or epithelial cell differentiation-related genes.

2. The method of claim 1, further comprising overexpressing and then reducing the desmosome-related genes or epithelial cell differentiation-related genes.

3. The method of claim 1, wherein the reprogramming factor is one or more factors selected from the group consisting of Oct4, Sox2, Klf4, and c-Myc.

4. The method of claim 1, comprising restricting the expression of the reprogramming factor.

5. The method of claim 1, wherein the desmosome-related gene or epithelial cell differentiation-related gene is at least one gene selected from among *Dsg3, Dsg4, Dsp, Evpl, Jup, Perp,* and *Pkpl.*

6. Pluripotent cells produced by the method for converting non-pluripotent cells into pluripotent cells of claim 1.

7. The pluripotent cells of claim 6, wherein the pluripotent cells exhibit reduced expression of at least one gene selected from the group consisting of *Nanog, Rex1,* and *Esrrb* compared to the corresponding genes in induced pluripotent stem cells (iPSCs).

8. The pluripotent cells of claim 6, wherein the pluripotent cells are possible to differentiate into cells constituting ectoderm, mesoderm, or endoderm.

9. The pluripotent cells of claim 6, wherein the pluripotent cells have reduced tumorigenicity compared to the iPSCs.

10. The pluripotent cells of claim 7, wherein the at least one gene exhibits reduced expression by at least 10 times compared to the corresponding gene in the iPSCs.

11. The pluripotent cells of claim 6, wherein the pluripotent cells have increased expression of Spink2 by at least 10 times compared to the iPSCs.

12. The pluripotent cells of claim 6, wherein the pluripotent cells further express at least one marker gene selected from the group consisting of Shisa3, Foxo4, Ptp4a3, Blvra, Mbnl3, Mthfd2, and Dctpp1.

13. Cells wherein:
the expression of a desmosome-related gene or epithelial cell differentiation-related gene is higher than the expression level of the corresponding gene in iPSCs; or
the expression level of *Spink2* gene is higher than the expression level of the gene in the iPSCs; and
the expression level of at least one gene selected from the group consisting of *Nanog, Rex1,* and *Esrrb* is reduced compared to the expression level of the corresponding gene in the iPSCs.

14. The cells of claim 13, wherein the cells, in which the expression of desmosome-related genes or epithelial cell differentiation-related genes is higher than the expression level of the corresponding gene in the iPSCs, express *Dsp* gene and *Shisa8* gene.

15. The cells of claim 13, wherein the cells, in which the expression level of *Spink2* gene is higher than the expression level of the gene in the iPSCs, exhibit pluripotency that is the potential to differentiate into any one of ectodermal cells, mesodermal cells, or endodermal cells.

16. A method for producing cells, the method comprising inducing the differentiation of the pluripotent cells of any one of claims 6 to 12, or cells of any one of claims 13 to 15.

17. A composition for cell transplantation or biological tissue regeneration, the composition comprising, as active ingredients:
the pluripotent cells of any one of claims 6 to 12;
the cells of any one of claims 13 to 15; or
the cells produced by the method for producing cells comprising inducing the differentiation of the pluripotent cells of any one of claims 6 to 12, or cells of any one of claims 13 to 15.

18. A method for evaluating the efficacy or toxicity of a substance to be tested, the method comprising bringing:
the pluripotent cells of any one of claims 6 to 12;
the cells of any one of claims 13 to 15; or
the cells produced by the method for producing cells comprising inducing the differentiation of the pluripotent cells of any one of claims 6 to 12, or cells of any one of claims 13 to 15, into contact with the substance to be tested.

19. A method for regenerating biological tissue by administering to an individual:
the pluripotent cells of any one of claims 6 to 12;
the cells of any one of claims 13 to 15; or
the cells produced by the method for producing cells comprising inducing the differentiation of the pluripotent cells of any one of claims 6 to 12, or cells of any one of claims 13 to 15.
